# EUROPEAN PATENT APPLICATION

(11) **EP 3 476 941 A1**
(43) Date of publication of application: **01.05.2019**
(21) Application number: 18153907.3
(22) Date of filing: 29.01.2018
(51) Int. Cl.: C12N 15/70

(54) **COMPOSITION FOR SOLUBILIZING TARGET PROTEIN AND USE THEREOF**

(30) Priority: 25.10.2017 KR 20170139497
(71) Applicant: Intelligent Synthetic Biology Center, Daejeon 34141 (KR)
(72) Inventor: Lee, Jun Hyoung, 34141 Daejeon (KR); Kim, Sun-Chang, 34141 Daejeon (KR); Geraldini, Almando, 34139 Daejeon (KR)
(74) Representative: Kuttenkeuler, David

(57) **Abstract**

Provided are a composition for solubilizing a target protein, including an expression cassette including a promoter and a gene encoding a fusion protein of a chaperone and an RNA-binding domain or an expression vector including the expression cassette; a transformant including the expression cassette or the expression vector; a kit including the composition or the transformant; and a method for producing the target protein by using the same.

## Description

### BACKGROUND OF THE INVENTION

### 1. Field of the Invention

The present invention relates to an expression cassette including a promoter and a gene encoding a fusion protein of a chaperone and an RNA-binding domain; an expression vector including the expression cassette; a composition for solubilizing a target protein, including the expression cassette or the expression vector; a transformant including the expression cassette or the expression vector; a kit including the composition or the transformant; and a method for producing the target protein by using the same.

### 2. Description of the Related Art

When recombinant proteins are produced in heterologous cells, there is a problem that recombinant proteins form insoluble aggregates, and thus there is a need for a method for increasing solubilization of recombinant proteins (Current opinion in biotechnology, 2001, 12, 202-207). However, a method for solubilizing recombinant proteins, which may be applied to various recombinant proteins, has not been developed yet.

Under this background, the present inventors have made many efforts to develop a method for increasing solubilization of a target protein, and as a result, they found that a chaperone-recruiting mRNA scaffold (CRAS) system and a chaperone-substrate co-localized expression (CLEX) system increase solubilization of various target proteins, thereby completing the present invention.

### SUMMARY OF THE INVENTION

An object of the present invention is to provide a composition for solubilizing a target protein, the composition including an expression cassette including a promoter and a gene encoding a fusion protein of a chaperone and an RNA-binding domain or an expression vector including the expression cassette.

Another object of the present invention is to provide a transformant comprising the expression cassette including the promoter and the gene encoding the fusion protein of the chaperone and the RNA-binding domain or the expression vector including the expression cassette.

Still another object of the present invention is to provide a kit for producing the target protein, the kit comprising the composition or the transformant.

Still another object of the present invention is to provide the expression cassette comprising the promoter and the gene encoding the fusion protein of the chaperone and the RNA-binding domain or the expression vector including the expression cassette.

Still another object of the present invention is to provide an expression cassette comprising a promoter, a gene encoding a target protein, and a gene of a hairpin structure at the 3'-terminus thereof, or an expression vector including the expression cassette.

Still another object of the present invention is to provide a method for producing a target protein, the method comprising the steps of:
(i) introducing into a microorganism (a) the expression cassette including the promoter and the gene encoding the fusion protein of the chaperone and the RNA-binding domain or the expression vector including the expression cassette and (b) the expression cassette including the promoter, the gene encoding the target protein, and the gene of the hairpin structure at the 3'-terminus thereof, or the expression vector including the expression cassette; and
(ii) culturing the microorganism.

Still another object of the present invention is to provide an expression cassette comprising (a) a cistron encoding a chaperone and (b) a cistron encoding a target protein, or an expression vector including the expression cassette.

Still another object of the present invention is to provide a composition for solubilizing a target protein, the composition comprising the expression cassette including (a) the cistron encoding the chaperone and (b) the cistron encoding the target protein, or the expression vector including the expression cassette.

Still another object of the present invention is to provide a transformant comprising the expression cassette including (a) the cistron encoding the chaperone and (b) the cistron encoding the target protein, or the expression vector including the expression cassette.

Still another object of the present invention is to provide a kit for producing the target protein, the kit comprising the expression cassette including (a) the cistron encoding the chaperone and (b) the cistron encoding the target protein; the expression vector including the expression cassette; the composition for solubilizing the target protein including the expression cassette or the expression vector; or the transformant including the expression cassette or the expression vector.

Still another object of the present invention is to provide a method for producing the target protein, the method comprising the steps of:
(i) preparing the transformant including the expression cassette including (a) the cistron encoding the chaperone and (b) the cistron encoding the target protein, or the expression vector including the expression cassette; and
(ii) culturing the transformant.

### BRIEF DESCRIPTION OF THE DRAWINGS

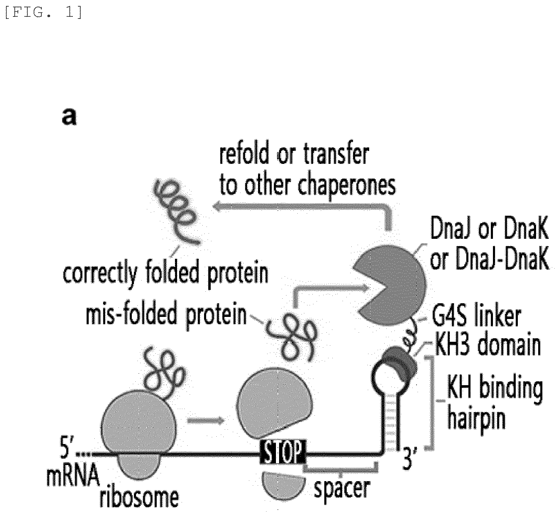

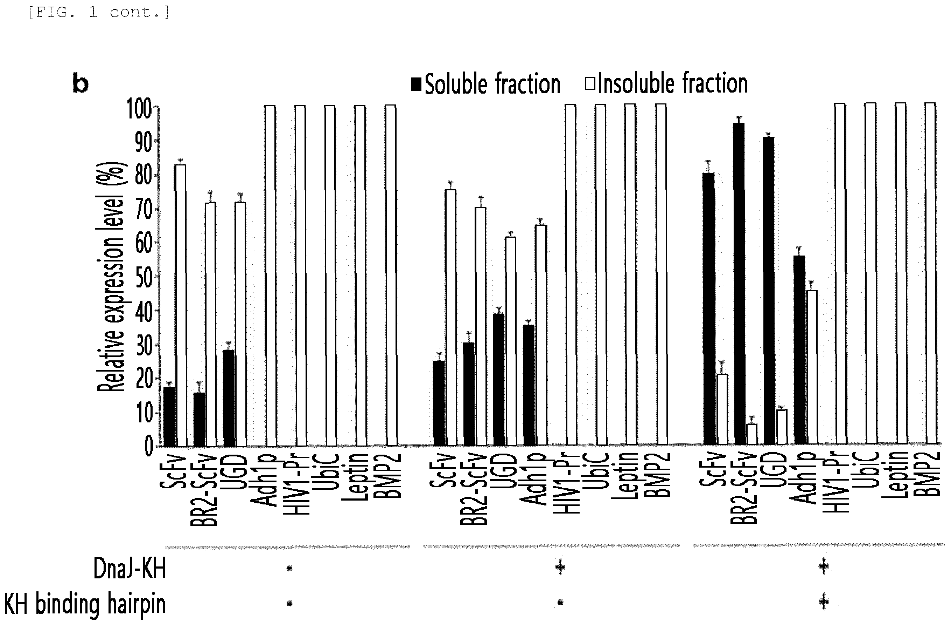

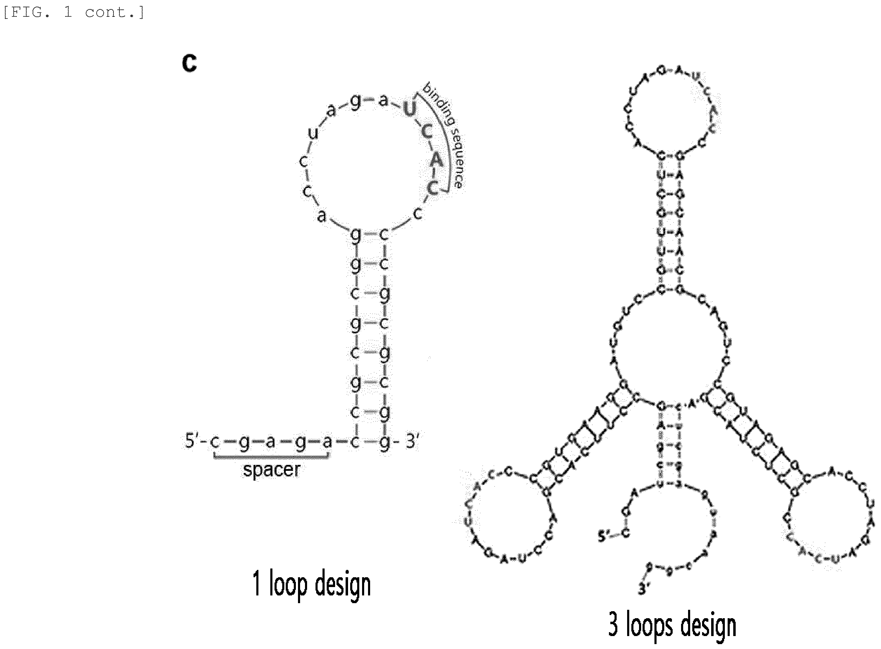

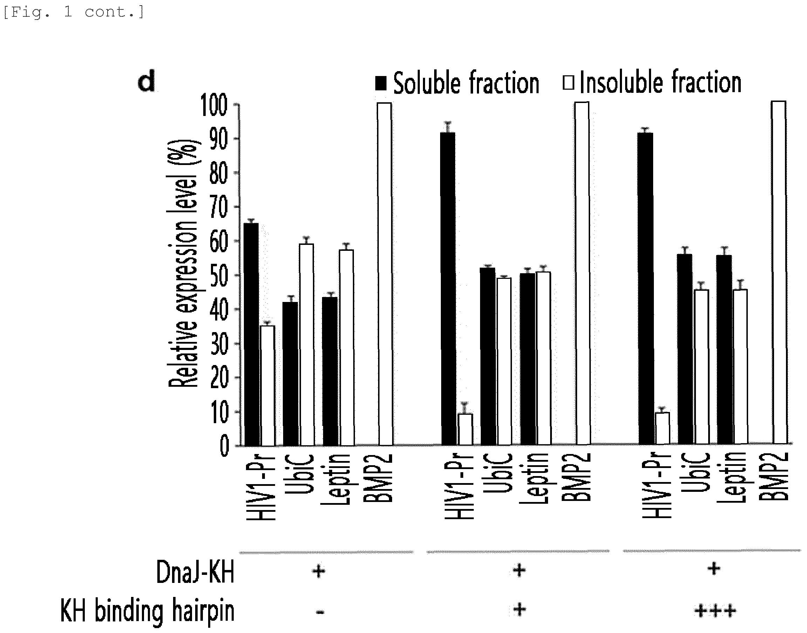

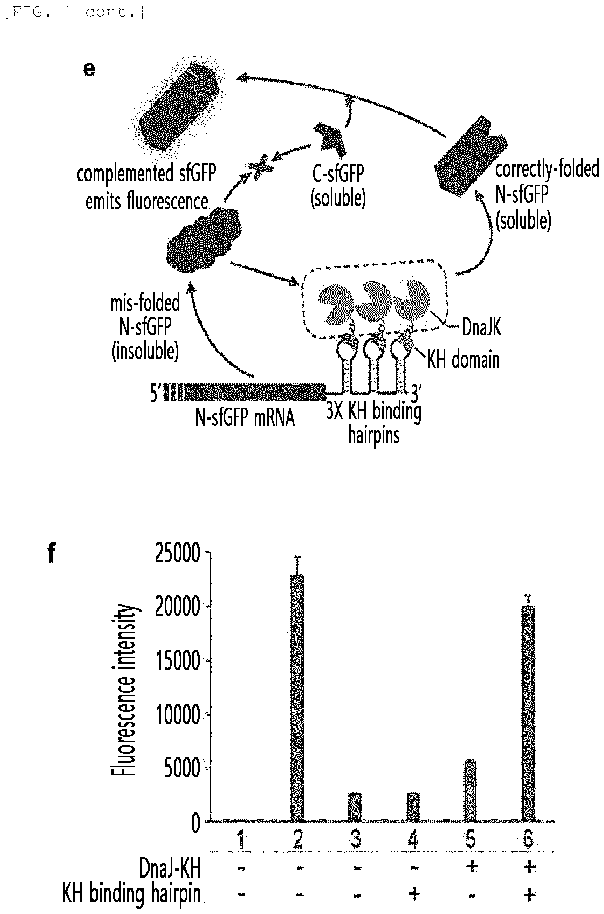

FIG. 1 shows solubilization of recombinant proteins by a chaperone-recruiting mRNA scaffold (CRAS) system:
   (A) is a schematic illustration of the CRAS system. A fusion protein of a chaperone molecule (DnaJ, DnaK, or a complex of DnaJ and DnaK (DnaJ-DnaK)) and an RNA-binding domain KH of Nova-1 was expressed together with a target recombinant protein to which a KH cognate stem-loop sequence was inserted as shown in (C). The chaperon complex binds with an initial protein to prevent protein aggregation or transfers the protein to other chaperone molecules for correct folding by introducing the KH-binding structure into the 3'UTR of mRNA;
   (B) shows the result of solubilization of eight selected aggregatable recombinant proteins (ScFv, HIV-1 protease, BR2-ScFv, UGD, Adhip, UbiC, Leptin, BMP2) when they were expressed with DnaJ-KH alone; or DnaJ-KH and 3'UTR hairpin loop;
   (D) shows the result of solubilization according to one(+) or three (+++) 3'UTR sequence(s), when four recombinant proteins which were not solubilized by the CRAS system using DnaJ-KH were applied to a CRAS system using a chimeric DnaJ-KH;
   (E) illustrates a split GFP assay to examine *in vivo* protein solubilization activity of the CRAS system; and
   (F) shows the result of *in vivo* solubilization of sfGFP N-terminus by the CRAS system. Fluorescence intensities of *E.coli* BL21(DE3) cells including (1) pET16b and pAMT7 (a negative control group); (2) pET16b-sfGFP and pAMT7-DnaJK-KH (a positive control group); (3) plasmid of sfGFP N-terminus and C-terminus in the absence of KH-binding hairpin and DnaJ-KH; (4) plasmid of sfGFP N-terminus and C-terminus in the presence of KH-binding hairpin and in the absence of DnaJ-KH; (5) plasmid of sfGFP N-terminus and C-terminus in the absence of KH-binding hairpin and in the presence of DnaJ-KH; or (6) plasmid of sfGFP N-terminus and C-terminus in presence of KH-binding hairpin and DnaJ-KH (CRAS system). Quantification of soluble ScFv was performed on SDS-PAGE images obtained by using an ImageJ v1.48 software (NHI, USA) through Coomassie blue staining. The error bars in (B), (D) and (F) represent ± standard deviations from three independent experiments.
FIG. 2 shows solubilization of ScFv over time after applying the CRAS system having one loop (A) or three loops (B) in *E.coli* BL21(DE3):
   lane M, marker(ELPIS, Daejeon, South Korea); lane W, whole cell lysate; lane S, soluble fraction; lane I, insoluble fraction.
FIG. 3 shows the result of solubilization according to the length of a spacer between the stop codon and the 3'UTR-binding loop in the CRAS system. When the CRAS system was applied (B), ScFv solubility was increased, as compared with single expression of ScFv (A), 4 hours after induction of expression:
   lane 1, in the absence of spacer between stop codon and 3'UTR-binding loop; lane 2, in the presence of 5-nt spacer between stop codon and 3'UTR-binding loop; lane 3, in the presence of 30-nt spacer between stop codon and 3'UTR-binding loop; lane M, marker; lane W, whole cell lysate; lane S, soluble fraction; lane I, insoluble fraction.

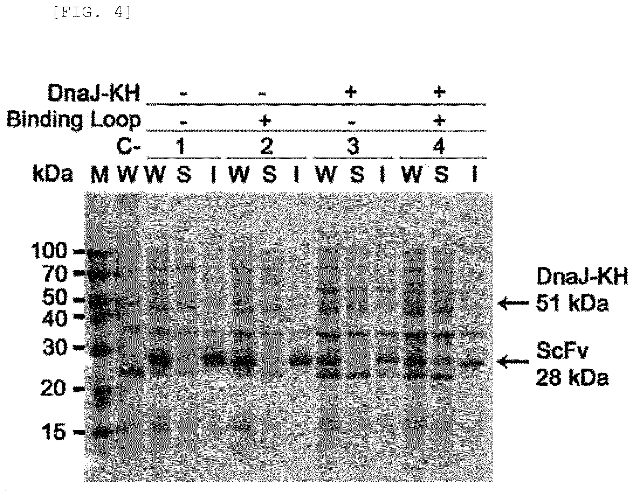

FIG. 4 shows increased solubility of ScFv by the CRAS system in a *dnaK* knockout strain (lane C-: whole cell lysate of *E.coli* BL21(DE3) having pET16b and pAMT7 (a negative control group), lane 1: in the absence of KH-binding hairpin and DnaJ-KH, lane 2: in the presence of KH-binding hairpin and in the absence of DnaJ-KH, lane 3: in the absence of KH-binding hairpin and in the presence of DnaJ-KH, lane 4: in the presence of KH-binding hairpin and DnaJ-KH (RNA Scaffold), lane M: marker, lane W: whole cell lysate, lane S: soluble fraction, lane I: insoluble fraction).

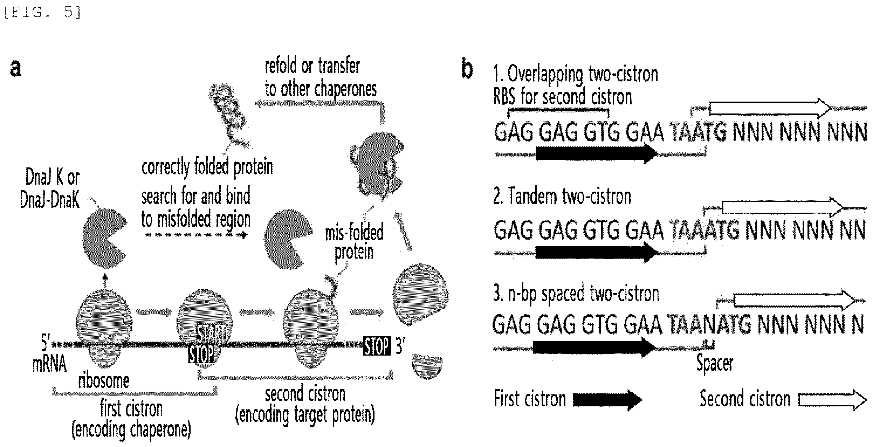

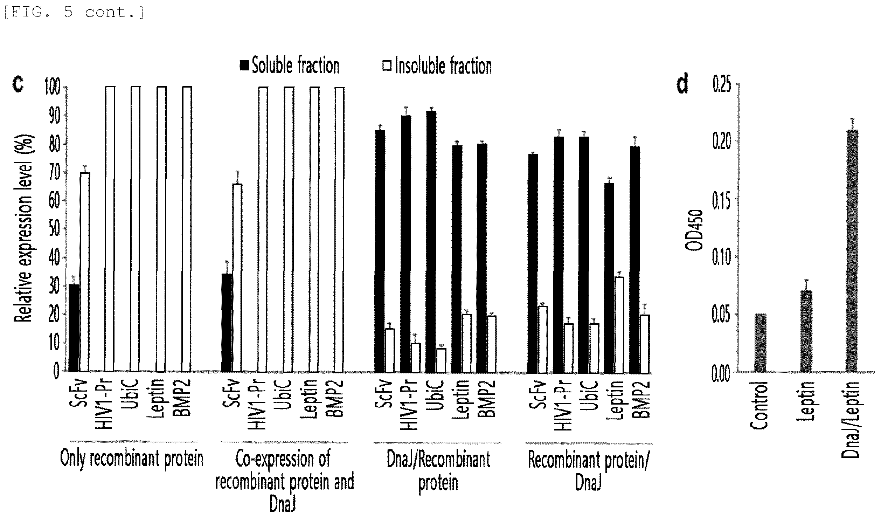

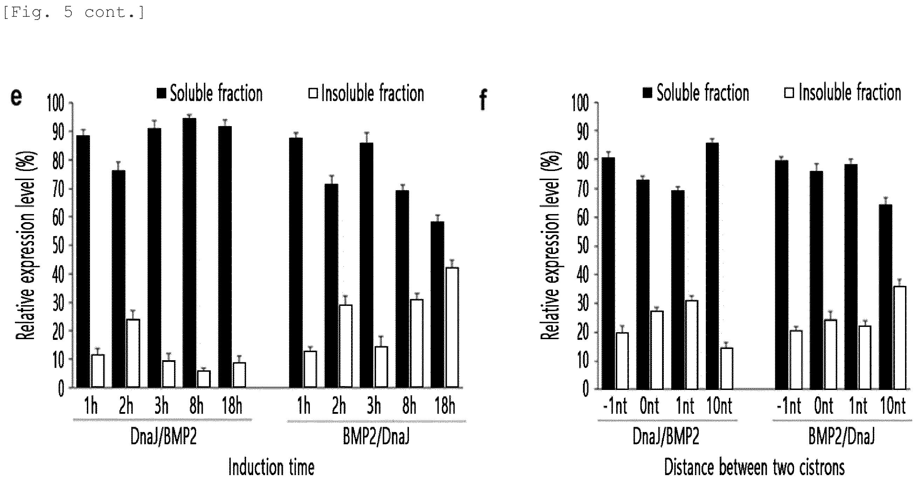

FIG. 5 shows solubilization of recombinant proteins by a CLEX system:
   (A) is a schematic illustration of the CLEX system. A translationally coupled two-cistron expression system was used to translate DnaJ and target protein in proximity, from a single mRNA transcript;
   (B) shows overlapping of the stop codon (TAA) of the first cistron with the start codon (ATG) of the second cistron, or a tandem arrangement thereof, or a space of n nt placed therebetween. 12 nucleotides in the 3'-sequence of the first cistron function as a ribosome-binding site (RBS) for the second cistron;
   (C) shows the result of solubilization of five aggregatable recombinant proteins when chaperone DnaJ was used as the first or second cistron in the CLEX system;
   (D) shows the result of measuring OD₄₅₀ of samples obtained from *E.coli* BL21(DE3) having the following plasmids in order to examine a level of correctly folded leptin: pET16b (a negative control group), pET16b-Leptin (leptin overexpression) and pET16b-DnaJ/Leptin (DnaJ and leptin overexpression in CLEX system, CLEX system);
   (E) shows the result of solubilization of BMP2 according to the order of DnaJ and target protein in the translationally coupled two-cistron expression system; and
   (F) shows the result of solubilization of BMP2 according to the distance between the cistrons in the translationally coupled two-cistron expression system;
   the slash (/) between the proteins represents the order of the genes encoding the respective proteins in mRNA. Relative expression levels were quantified by using an ImageJ v1.48 software (NJI, USA), based on the result of SDS-PAGE. The error bars in (C), (D), (E), and (F) represent ± standard deviations from three independent experiments.

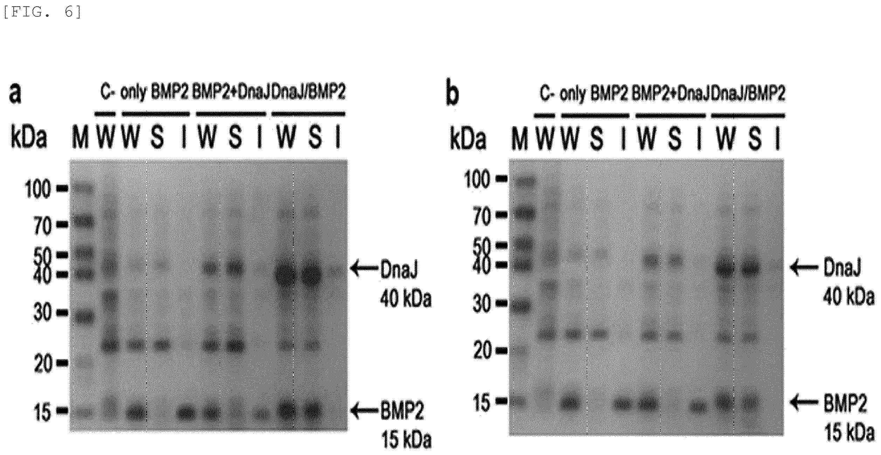

FIG. 6 shows increased solubilization of BMP2 in *dnaJ* knockout strain (A) or *dnaK* knockout strain (B) by the CLEX system (lane C-: whole cell lysate of *E.coli* BL21(DE3) having pET16b and pAMT7 (a negative control group), lane only BMP2: BMP2 expression in the absence of DnaJ, lane BMP2+DnaJ: BMP2 expression in the presence of DnaJ, lane DnaJ/BMP2: DnaJ placed in the first cistron and BMP2 placed in the second cistron in the CLEX system, lane M: marker, lane W: whole cell lysate, lane S: soluble fraction, lane I: insoluble fraction).

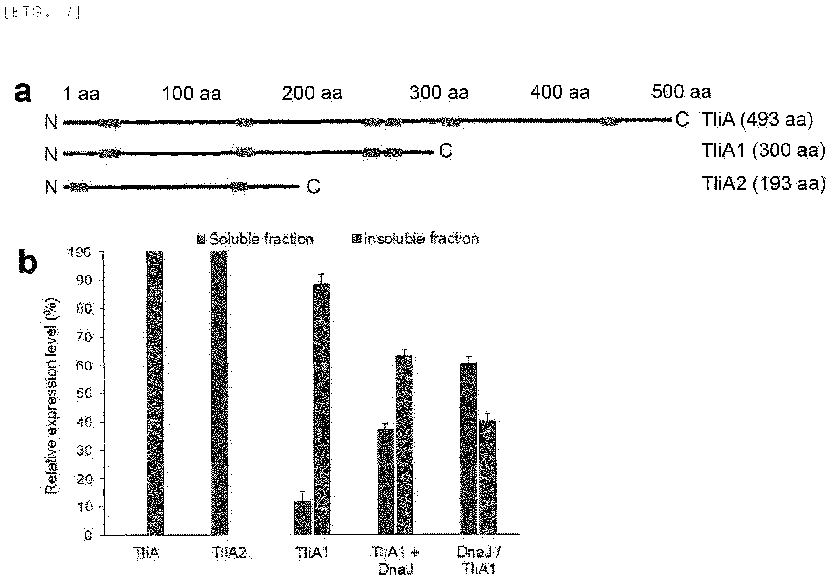

FIG. 7 shows the result of solubilization according to the size of recombinant protein in the CLEX system:
   (A) shows distribution of a DnaJ/DnaK-binding sequence in lipase TliA, indicated by red rectangular blocks. The binding site was predicted by using a Limbo chaperone binding site predictor; and
   (B) shows the result of solubilization of TliA1 fragment by the CLEX system. TliA1 + DnaJ represents simple coexpression of TliA and DnaJ, DnaJ/TliA1 represents CLEX system using DnaJ as the first cistron. Relative expression levels were quantified by using an ImageJ v1.48 software (NJI, USA), based on the result of SDS-PAGE. The error bars in (B) represent ± standard deviations from three independent experiments.

### DETAILED DESCRIPTION OF THE PREFERRED EMBODIMENTS

To achieve the above objects, an aspect of the present invention provides a composition for solubilizing a target protein, the composition including an expression cassette including a promoter and a gene encoding a fusion protein of a chaperone and a RNA-binding domain, or an expression vector including the expression cassette.

The fusion protein expressed by the expression cassette or the expression vector binds to mRNA including a sequence of the target protein via the RNA-binding domain, and as a result, the chaperone of the fusion protein exists near the translated target protein, leading to correct folding of the target protein. That is, the fusion protein may increase solubility of the target protein in a general manner, irrespective of the kind of the target protein. In this case, mRNA including the sequence of the target protein may have a hairpin structure (including an RNA site to which the RNA-binding domain of the fusion protein binds) at 3'-UTR in order to bind with the fusion protein.

As used herein, the term "chaperone" refers to a protein involved in folding and unfolding or assembly and disassembly of other proteins. Specifically, the chaperone of the present invention functions to induce or help folding of the target protein, thereby increasing solubility of the target protein. In the present invention, the chaperone, in the form of the fusion protein with the RNA-binding domain, binds to mRNA including the sequence of the target protein to help folding of the target protein near the translated target protein.

In the present invention, the chaperone is not limited to an origin (e.g., derived from a microorganism) and a specific sequence thereof, as long as it is involved in folding of the target protein. Specifically, the chaperone may be a protein derived from prokaryotes (e.g., bacteria, etc.), eukaryotes, or archaea, and a sequence thereof may be obtained from a known database, GenBank of NCBI, etc. Specifically, the chaperone may be DnaJ, Dnak, GroEL, GroES, HtpG, ClpA, ClpX, or ClpP, and more specifically, DnaJ or Dnak. The DnaJ or Dnak may have an amino acid sequence encoded by a nucleotide sequence of SEQ ID NO: 74 or 75, but is not limited thereto. Further, the chaperone may be Hsp40, Hsp60, Hsp70, Hsp90, Hsp100, or Hsp104.

In the present invention, the chaperone may be in a combination of two or more of the same or different kind of chaperone units. Specifically, the chaperone may be DnaJK in which DnaJ and Dnak fuse to each other, but is not limited thereto.

As used herein, the term "RNA-binding domain (RBD)" refers to a domain binding to RNA. Specifically, the RNA-binding domain of the present invention forms a complex or fusion protein, together with the chaperone, such that the complex or fusion protein including the chaperone binds to mRNA including the sequence of the target protein. That is, the RNA-binding domain locates the chaperone of the fusion protein near the target protein to be translated by ribosomes, leading to correct folding of the target protein through the adjacent chaperone.

In the present invention, the RNA-binding domain is not limited to an origin (e.g., derived from a microorganism) and a specific sequence thereof, as long as it binds to mRNA including the target protein. Specifically, the RNA-binding domain may be K Homology (KH) domain, bacteriophage MS2 coat protein, bacteriophage PP7 coat protein, sterile alpha motif (SAM), or RNA recognition motif (RRM), and a sequence thereof may be obtained from a known database, GenBank of NCBI, etc. Further, the KH domain may have an amino acid sequence encoded by a nucleotide sequence of SEQ ID NO: 76, but is not limited thereto.

In the present invention, the fusion protein may include one or more chaperones and the RNA-binding domain. Further, in the fusion protein, the chaperone may be linked to the N-terminus; the C-terminus; or the N-terminus and C-terminus of the RNA-binding domain, directly or via a linker. In the fusion protein, the RNA-binding domain may be linked to the N-terminus; the C-terminus; or the N-terminus and C-terminus of the chaperone, directly or via a linker.

The linker is not particularly limited, as long as it allows the chaperone and the RNA-binding domain of the fusion protein to exhibit activity. Specifically, the chaperone and the RNA-binding domain may be linked by using an amino acid such as glycine, alanine, leucine, isoleucine, proline, serine, threonine, asparagine, aspartic acid, cysteine, glutamine, glutamic acid, lysine, argininic acid, etc., and more specifically, by using several amino acids such as valine, leucine, aspartic acid, glycine, alanine, proline, etc. Much more specifically, for easy genetic manipulation, 1 to 10 amino acids of glycine, valine, leucine, aspartic acid, etc. may be linked and used. For example, in the present invention, the fusion protein was prepared by linking the C-terminus of the chaperone to the N-terminus of the RNA-binding domain via a G4S linker.

The fusion protein may include a polypeptide having a sequence in which one or more amino acid residues are different from those of an amino acid sequence of the wild-type of each protein or domain included in the fusion protein. Amino acid exchanges in proteins and polypeptides, which do not generally alter the activity of molecules, are known in the art. The most commonly occurring exchanges are Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, Asp/Gly, in both directions. The fusion protein may also include proteins with increased structural stability against heat, pH, etc. or increased activities by mutation or modification of the amino acid sequences.

The fusion protein or the polypeptide constituting the fusion protein may be prepared by a chemical peptide synthetic method known in the art, or prepared by amplifying the gene encoding the fusion protein by polymerase chain reaction (PCR) or synthesizing the gene by a known method and then cloning the gene into an expression vector to express the gene.

In a specific embodiment of the present invention, the fusion protein may have a form (DnaJK-KH), in which DnaJ and Dnak (DnaJK) as the chaperones and the KH domain as the RNA-binding domain bind to each other, but is not limited thereto.

The protein of the present invention may include not only the amino acid sequence encoded by the nucleotide sequence of each SEQ ID NO., but also an amino acid sequence having 80% or more, specifically 90% or more, more specifically 95% or more, much more specifically 97% or more sequence homology with the above sequence. Any amino acid sequence having the homology may be included without limitation, as long as it is an amino acid sequence of a protein having an efficacy that is substantially identical or corresponding to that of the above protein. It is apparent that an amino acid sequence having a deletion, modification, substitution, or addition of some sequence may be also within the scope of the present invention, as long as the amino acid sequence has the homology.

Furthermore, the gene encoding the protein of the present invention may include not only the nucleotide sequence of each SEQ ID NO., but also a nucleotide sequence having 80% or more, specifically 90% or more, more specifically 95% or more, much more specifically 98% or more, and most specifically 99% or more sequence homology with the above sequence, as long as it is a nucleotide sequence encoding a protein having an efficacy that is substantially identical or corresponding to that of the above protein. It is apparent that a nucleotide sequence having a deletion, modification, substitution, or addition of some sequence may be also within the scope of the present invention, as long as the nucleotide sequence has the homology.

As used herein, the term "homology" is intended to indicate the degree of similarity of a nucleotide sequence of a gene encoding a protein or an amino acid sequence, and if homology is sufficiently high, an expression product of the corresponding gene has the same or similar activity.

As used herein, the term "promoter" refers to a nucleotide sequence that regulates expression of another nucleotide sequence operably linked thereto in appropriate host cells, and as used herein, "operably linked" refers to a functional linkage between a nucleotide expression control sequence and a nucleotide sequence encoding a target protein or RNA in such a manner as to allow general functions. For example, a promoter and a nucleotide sequence encoding a protein or RNA are operably linked to influence expression of a coding sequence. A method for operably linking the promoter and the nucleotide sequence encoding the protein or RNA may be performed by a genetic recombinant technique well known in the art, and site-specific DNA cleavage and ligation may be achieved by using enzymes generally known in the art.

As used herein, the term "expression vector" refers to a DNA construct including the expression cassette, which is operably linked to an appropriate regulatory sequence to express the target protein in an appropriate host. The regulatory sequence may include a promoter capable of initiating transcription, an arbitrary operator sequence for regulating transcription, a sequence encoding an appropriate mRNA ribosome binding site, and sequences for regulating the termination of transcription and translation. Once a vector is transformed into an appropriate host, the vector may replicate and function independently of the host genome, or it may be integrated into the genome itself.

As long as the expression vector used in the present invention is replicable in host cells, any vector known in the art may be used without particular limitations. Example of the vector commonly used may include a natural or recombinant plasmid, cosmid, virus, and bacteriophage. Example of the phage vector or the cosmid vector may include pWE15, M13, MBL3, MBL4, IXII, ASHII, APII, t10, t11, Charon4A, Charon21A, etc. The plasmid vector may include pBR type, pUC type, pBluescriptII type, pGEM type, pTZ type, pCL type, pET type, pCP type etc. Specifically, pDZ, pACYC177, pACYC184, pCL, pECCG117, pUC19, pBR322, pMW118, pCC1BAC, pCP22, pAMT7, pET16b vector, etc. may be used, but is not limited thereto.

The vector usable in the present application is not particularly limited, and any known expression vector may be used. Also, the polynucleotide encoding the target protein may be inserted into the chromosome by a vector for chromosomal gene insertion. The insertion of the polynucleotide into the chromosome may be achieved by any method known in the art, for example, by homologous recombination, but is not limited thereto. A selection marker for confirming the chromosomal insertion may be further included. The selection marker is used to select a cell transformed with the vector, i.e., to confirm the insertion of the target nucleotide molecule. Markers that provide selectable phenotypes, such as drug resistance, auxotrophy, resistance to cytotoxic agents, or surface protein expression, may be used. Only cells expressing the selection marker are able to survive or to show different phenotypes under the environment treated with the selective agent, and thus the transformed cells may be selected.

The composition of the present invention may further include an expression cassette including a promoter, a gene encoding a target protein, and a gene of a hairpin structure at the 3'-terminus thereof, or an expression vector including the expression cassette. The promoter and the expression vector are the same as described above.

mRNA transcribed by the expression cassette or the expression vector has RNA of the target protein and the hairpin structure at 3'-UTR thereof, and the hairpin and the RNA-binding domain of the fusion protein may bind to each other. By this binding, a scaffold, in which the chaperone region of the fusion protein is recruited to 3'-UTR of mRNA including the sequence of the target protein, is formed. In the present invention, this scaffold is called chaperone-recruiting mRNA scaffold (CRAS).

As used herein, the term "hairpin" means a structure consisting of a doublestranded stem and a loop, which is formed by internal hydrogen bonding between bases of single-stranded DNA or RNA. The hairpin is used interchangeably with stem-loop. A sequence of the hairpin may be any sequence known in the art.

Further, the hairpin structure may have an RNA sequence (site), to which the RNA-binding domain of the fusion protein, i.e., the RNA-binding domain linked to the chaperone binds. Therefore, the gene of the hairpin structure included in the expression cassette or expression vector may include a sequence transcribing the RNA sequence to which the RNA-binding domain binds.

In the present invention, the expression cassette or expression vector may further include a spacer gene between the gene encoding the target protein and the gene of the hairpin structure. The spacer gene refers to a non-coding gene between the genes, and any sequence known in the art may be used.

In the present invention, the gene of the hairpin structure may have one or more of the gene of the hairpin structure. In this case, one or more of the hairpin, to which the fusion protein of the present invention binds, may exist in mRNA including the sequence of the target protein, and therefore, a larger number of chaperones may be recruited to the mRNA.

One or more of the gene of the hairpin structure may include a spacer gene between hairpin structures, but is not limited thereto.

In the present invention, the expression cassette or expression vector may further include a ribosome-binding site. The "ribosome-binding site (RBS)" refers to a ribosome-binding site that allows mRNA transcribed from DNA to bind with ribosomes in host cells during initiation of protein biosynthesis. The ribosome-binding site may be any sequence known in the art.

Another aspect of the present invention provides a transformant including the expression cassette including the promoter and the gene encoding the fusion protein of the chaperone and the RNA-binding domain or the expression vector including the expression cassette. Specifically, the transformant may further include the expression cassette including the promoter, the gene encoding the target protein, and the gene of the hairpin structure at the 3'-terminus thereof, or the expression vector including the expression cassette.

The promoter, the chaperone, the RNA-binding domain, the fusion protein, the gene of the hairpin structure, the expression cassette, and the expression vector are the same as described above.

The transformant may produce the target protein with improved solubility by CRAS system.

As used herein, the term "transformation" means that the expression cassette of the present invention or the expression vector including the expression cassette is introduced into a host cell in such a way that the protein encoded the polynucleotide of the expression cassette or the expression vector is expressed in the host cell. As long as the transformed polynucleotide may be expressed in the host cell, it may be either integrated into and placed in the chromosome of the host cell, or exist extrachromosomally. Further, the polynucleotide may include DNA and RNA encoding the target protein. The polynucleotide may be introduced in any form, as long as it may be introduced into the host cell and expressed therein. For example, the polynucleotide may be introduced into the host cell in the form of an expression cassette, which is a gene construct including all elements required for its autonomous expression. Generally, the expression cassette may include a promoter operably linked to the polynucleotide, transcriptional termination signals, ribosome binding sites, or translation termination signals. The expression cassette may be in the form of a self-replicable expression vector. Also, the polynucleotide as it is may be introduced into the host cell and operably linked to sequences required for expression in the host cell, but is not limited thereto. Any transformation method may be employed, as long as it is used to introduce a nucleotide into a host cell. Depending on the host cell, a suitable standard technique may be selected as known in the art. For example, electroporation, calcium phosphate (CaPO₄) precipitation, calcium chloride (CaCl₂) precipitation, microinjection, a polyethyleneglycol (PEG) technique, a DEAE-dextran technique, a cationic liposome technique, and a lithium acetate DMSO technique may be used, but is not limited thereto.

The transformant is not limited, as long as the protein encoded by the polynucleotide of the expression cassette or expression vector of the present invention may be expressed therein. The transformant may be specifically a plant cell, a bacterial cell, a fungal cell, a yeast cell, or an animal cell, and more specifically, a microorganism of the genus *Escherichia.*

Still another aspect provides a kit for producing the target protein, the kit including the composition for solubilizing the target protein or the transformant.

Still another aspect provides the expression cassette including the promoter and the gene encoding the fusion protein of the chaperone and the RNA-binding domain, or the expression vector including the expression cassette.

The promoter, the chaperone, the RNA-binding domain, the fusion protein, the expression cassette, and the expression vector are the same as described above.

Still another aspect provides the expression cassette including the promoter, the gene encoding the target protein, and the gene of the hairpin structure at the 3'-terminus thereof, or the expression vector including the expression cassette.

The promoter, the gene of the hairpin structure, the expression cassette, and the expression vector are the same as described above.

Still another aspect provides a kit for producing the target protein, the kit including the composition or the transformant.

Still another aspect provides a method for producing the target protein, the method including the steps of:
(i) preparing a transformant including (a) the expression cassette including the promoter and the gene encoding the fusion protein of the chaperone and the RNA-binding domain or the expression vector including the expression cassette and (b) the expression cassette including the promoter, the gene encoding the target protein, and the gene of the hairpin structure at the 3'-terminus thereof, or the expression vector including the expression cassette; and
(ii) culturing the transformant.

The promoter, the chaperone, the RNA-binding domain, the fusion protein, the gene of the hairpin structure, the expression cassette, the expression vector, and the transformant are the same as described above.

The step (i) of preparing the transformant may be a step of additionally introducing (a) the expression cassette including the promoter and the gene encoding the fusion protein or the expression vector including the expression cassette into a host cell previously having (b) the expression cassette including the promoter, the gene encoding the target protein, and the gene of the hairpin structure at the 3'-terminus thereof, or the expression vector including the expression cassette, but is not limited thereto.

Further, the step (i) of preparing the transformant may be a step of additionally introducing (b) the expression cassette including the promoter, the gene encoding the target protein, and the gene of the hairpin structure at the 3'-terminus thereof, or the expression vector including the expression cassette into a host cell previously having (a) the expression cassette including the promoter and the gene encoding the fusion protein or the expression vector including the expression cassette, but is not limited thereto.

Furthermore, the step (i) of preparing the transformant may be a step of introducing into the host cell (a) the expression cassette including the promoter and the gene encoding the fusion protein or the expression vector including the expression cassette and (b) the expression cassette including the promoter, the gene encoding the target protein, and the gene of the hairpin structure at the 3'-terminus thereof, or the expression vector including the expression cassette sequentially, simultaneously, or reversely, but is not limited thereto.

In the production method, the step of (ii) culturing the transformant may be performed by, but is not particularly limited to, a known batch, continuous, or fed-batch culture method. In this regard, culture conditions may be, but are not particularly limited to, maintained at optimal pH (e.g., pH 5 to pH 9, specifically pH 6 to pH 8, and most specifically pH 6.8) by using basic compounds (e.g., sodium hydroxide, potassium hydroxide, or ammonia) or acidic compounds (e.g., phosphoric acid or sulfuric acid) and at an aerobic condition by adding oxygen or oxygen-containing gas mixture to a cell culture. The culture temperature may be maintained at 20°C to 45°C, and specifically at 25°C to 40°C, and cultured for about 10 hours to about 160 hours, but is not limited thereto. The target protein produced by the culturing may be secreted to a culture medium or may remain within cells.

Further, the culture medium to be used may include sugars and carbohydrates (e.g., glucose, sucrose, lactose, fructose, maltose, molasses, starch, and cellulose), oils and fats (e.g., soybean oil, sunflower seed oil, peanut oil, and coconut oil), fatty acids (e.g., palmitic acid, stearic acid, and linoleic acid), alcohols (e.g., glycerol and ethanol), and organic acids (e.g., acetic acid) as a carbon source individually or in combination, but is not limited thereto. As a nitrogen source, nitrogen-containing organic compounds (e.g., peptone, yeast extract, beef stock, malt extract, corn steep liquor, soybean meal powder, and urea), or inorganic compounds (e.g., ammonium sulfate, ammonium chloride, ammonium phosphate, ammonium carbonate, and ammonium nitrate) may be used individually or in combination, but is not limited thereto. As a phosphorus source, potassium dihydrogen phosphate, dipotassium phosphate, or sodium-containing salt corresponding thereto may be used individually or in combination, but is not limited thereto. The culture medium may include other essential growth-stimulating substances such as metal salts (e.g., magnesium sulfate or iron sulfate), amino acids, and vitamins.

The production method may further include the step of collecting the target protein from the transformant or the culture thereof. The method for collecting the target protein produced in the culturing step may be performed by an appropriate method known in the art according to the culturing method, thereby collecting the target protein from the transformant or the culture thereof. For example, cell lysis, centrifugation, filtration, anion exchange chromatography, crystallization, HPLC, etc. may be used. The target protein may be collected from the transformant or the culture thereof by an appropriate method known in the art.

Still another aspect provides a method for solubilizing a target protein, the method including the steps of:
(i) preparing a transformant including (a) the expression cassette including the promoter and the gene encoding the fusion protein of the chaperone and the RNA-binding domain or the expression vector including the expression cassette and (b) the expression cassette including the promoter, the gene encoding the target protein, and the gene of the hairpin structure at the 3'-terminus thereof, or the expression vector including the expression cassette; and
(ii) culturing the transformant.

The promoter, the chaperone, the RNA-binding domain, the fusion protein, the gene of the hairpin structure, the expression cassette, the expression vector, the transformant, step (i), and step (ii) are the same as described above.

Still another aspect of the present invention provides an expression cassette including (a) a cistron encoding a chaperone and (b) a cistron encoding a target protein, or an expression vector including the expression cassette.

The chaperone, the expression cassette, and the expression vector are the same as described above.

Both the chaperone and the target protein are translated by the expression cassette or the expression vector, and as a result, the chaperone may increase correct folding of the target protein. In the present invention, the system is called a CLEX (chaperone-substrate co-localized expression) system.
(a) and (b) may be connected in either sequential or reverse order from 5'- to 3'-direction, and the stop codon of (a) or (b) may be overlapped with the start codon of (b) or (a), but is not limited thereto.

Still another aspect of the present invention provides a composition for solubilizing the target protein, the composition including the expression cassette including (a) the cistron encoding the chaperone and (b) the cistron encoding the target protein, or the expression vector including the expression cassette.

The chaperone, the expression cassette, and the expression vector are the same as described above.

Still another aspect of the present invention provides a transformant including the expression cassette including (a) the cistron encoding the chaperone and (b) the cistron encoding the target protein, or the expression vector including the expression cassette.

The chaperone, the expression cassette, the expression vector, and the transformant are the same as described above.

Still another aspect of the present invention provides a kit for producing the target protein, the kit including the expression cassette including (a) the cistron encoding the chaperone and (b) the cistron encoding the target protein; the expression vector including the expression cassette; the composition for solubilizing the target protein including the expression cassette or the expression vector; or the transformant including the expression cassette or the expression vector.

The chaperone, the expression cassette, the expression vector, and the transformant are the same as described above.

Still another aspect of the present invention provides a method for producing the target protein, the method including the steps of:
(i) preparing the transformant including the expression cassette including (a) the cistron encoding the chaperone and (b) the cistron encoding the target protein, or the expression vector including the expression cassette; and
(ii) culturing the transformant.

The chaperone, the expression cassette, the expression vector, the transformant, the culturing, and the production method are the same as described above.

The production method may further include the step of collecting the target protein from the transformant or the culture thereof.

Hereinafter, the construction and effect of the present invention will be described in more detail with reference to Examples. However, these Examples are for illustrative purposes only, and the scope of the present invention is not intended to be limited by these Examples.

### Experimental Example 1. Microorganisms, enzymes, and chemicals

*E.coli* XL1-Blue (Stratagene, La Jolla, CA, USA) and BL21(DE3) (Novagen, Madison, WI, USA) strains were used. XL-1 Blue strain was used for general cloning and BL21(DE3) strain was used for gene expression. A strain free of *dnaJ* and *dnaK* genes was prepared by known Pi transduction (Current protocols in molecular biology, 2007, Chapter 1, Unit1 17). BW25113 strains having single knock-outs (*ΔdnaJ* and *ΔdnaK*) were obtained from Keio collection, and used as a donor strain for BL21(DE3). Colony PCR was performed to screen deletion strains by using primer pairs around a target gene (Table 1). Then, a kanamycin resistance cassette was removed from the integration region in the host genome by using a pCP22 plasmid-derived FLP recombinant enzyme. Oligonucleotides (Genotech, Daejeon, Korea) and genes (Bioneer, Daejeon, Korea) used in the present invention are described in Table 1. Chemicals were purchased from Sigma-Aldrich (St. Louis, MO, USA).

**[Table 1]**

| **Proteins/ genes** | **Plasmid** | SEQ ID NO. | **Primers (5' to 3')** |
|---|---|---|---|
| **DnaJ (NcoI/XhoI)** | pAMT7-DnaJ (PT7::*dnaJ*; pBR322 ori, Cm^{R}) | **1** | **DnaJNcoF:** 5'-TGATAACCATGGAAGATTCTACGGTTAACACAATGG CTAAGCAAGATTATTACG-3' |
| | | **2** | **DnaJXhoR:** 5'-ATTTCACTCGAGGCGGGTCAGGTCGTCAAA-3' |
| **KH RNA binding domain (NcoI/XhoI)** | pAMT7-KH (PT7::*KH*; pBR322 ori, Cm^{R}) | **3** | **KHNcoF1:** 5'-TAATGACCATGGAAACCGACGGTTCTAAAGACGTTGT TGAAATCGCTGTTCCGGAAAACCTGGTTGGTGCTATCCTGGGTAA AGGTGGTAAAAC-3' |
| | | **4** | **KHRecR1:** 5'-GCCCGGAACAAATTCACCTTTTTTAGAAATCTGGATA CGAGCACCTGTCAGTTCCTGGTATTCAACCAGGGTTTTACCACCTT TACCCAGGA-3' |
| | | **5** | **KHRecF2:** 5'-AAAAGGTGAATTTGTTCCGGGCACCCGTAACCGTAA AGTTACCATCACAGGCACCCCGGCTGCTACCCAGGCTGCTCAGTA CCTGATCACAC-3' |
| | | **6** | **KHXhoR2** 5'-TTATCACTCGAGTTAACCAACTTTCTGCGGGTTAGCA GCACGAACACCCTGTTCGTAGGTGATACGCTGTGTGATCAGGTAC TGAGCAGC-3' |
| **DnaJ-KH (NcoI/XhoI)** | pAMT7-DnaJ-K H (PT7::*dnaJ*-*KH*; pBR322 ori, Cm^{R}) | **1** | **DnaJNcoF:** 5'-TGATAACCATGGAAGATTCTACGGTTAACACAAT GGCTAAGCAAGATTATTACG-3' |
| | | **7** | **DnaJLinkR:** 5'- GCTGCCGCCACCACCGCTACCGCCACCGCCGCG GGTCAGGTCGTCAAA-3' |
| | | **8** | **LinkKHF:** 5'-GGTAGCGGTGGTGGCGGCAGCACCGACGGTTCTAAA GACGTT-3' |
| | | **9** | **KHXhoR:** 5'-ATTTCACTCGAGTTAACCAACTTTCTGCGGGTT-3' |
| **DnaJ-KH-6xH is (NcoI/EcoRI)** | pAMT7-DnaJ-K H-6xHis (PT7::*dnaJ*-*KH*; pBR322 ori, Cm^{R}) | **1** | **DnaJNcoF:** 5'-TGATAACCATGGAAGATTCTACGGTTAACACAAT GGCTAAGCAAGATTATTACG-3' |
| | | **10** | **KH-6xHis-EcoR:** 5'-CGATTAGGATCCTCATCATTAATGATGGTGGTG ATGGTGAGATCCACGCGGAACCAGACCAACTTTCTGCGGGTTAG-3' |
| **DnaJK-KH (NcoI/XhoI)** | pAMT7-DnaJK-KH | **1** | **DnaJNcoF:** 5'- TGATAACCATGGAAGATTCTACGGTTAACACAAT GGCTAAGCAAGATTATTACG-3' |
| | (PT7::*dnaJ-dna K-KH*; pBR322 ori, Cm^{R}) | **11** | **DnaJR:** 5'-AGAACCTCCGCCGCCAGAACCCCCGCCACCGCGGGTC AGGTCGTCAAAAAA-3' |
| | | **12** | **DnaKF:** 5'-_TCTGGCGGCGGAGGTTCTGGTAAAATAATTGGTATCGA CCTGG-3' |
| | | **13** | **DnaKR** 5'-_GCTACCGCCACCGCCTTTTTTGTCTTTGACTTCTTCAAA TTC-3' |
| | | **14** | **KHF** 5'-GACAAAAAAGGCGGTGGCGGTAGC-3' |
| | | **9** | **KHXhoR:** 5'-TTTCCAGAACTCGAGTTAACCAACTTTCTGCGGGT-3' |
| **ScFv (NdeI/XhoI)** | pET16b-ScFv (PT7::*scFv*; pBR322 ori, Amp^{R}) | **15** | **ScFvNdeF:** 5'-TGATAACATATGCAGGTCCAACTGCAGC-3' |
| | | **16** | **ScFvXhoR:** 5'-TCATTACTCGAGTCATCATTAGTGGTGGTGGTGGTG GTGTTTGATCTCCAGCTTGGTCC-3' |
| ***scfv* with 1x binding loop (NdeI/XhoI)** | pET16b-ScFv1L (pT7::*scFv*-*(loop )₁*; pBR322 ori, Amp^{R}) | **15** | **ScFvNdeF.** 5'-TGATAACATATGCAGGTCCAACTGCAGC-3' |
| | | **17** | **ScFv1LXhoR:** 5'-CCGTTACTCGAGCCGCGCGGGGTGATCTAGGTC CGCGCGGTCGTCGTCGTCATCATTAGTGGTGGTGGTGGTGGTGTT TGATCTCCAGCTTGGTCC-3' |
| ***scfv* with 3x binding loop (NdeI/XhoI)** | pET16b-ScFv3L (PTT::*scFv*-*(loop* )*₃*; pBR322 ori, Amp^{R}) | **15** | **ScFvNdeF:** 5'-TGATAACATATGCAGGTCCAACTGCAGC-3' |
| | | **18** | **3L-1R:** 5'-AGGTGAGCAACGGACATCCTTCACGGGTGATCTAGGTC GTGAAGGCTCGATCGTCATCATTAGTGGTGGTGGTGGTGGTG-3' |
| | | **19** | **3LXhoR:** 5'-CCGTTACTCGAGTCGTAGAGCGGTGATCTAGGTGCTC TACGGACTGCGTTGCTCGGTGATCTAGGTGAGCAACGGACATCCT TCACG-3' |
| **BR2-ScFv (NdeI/XhoI)** | pET16b-BR2Sc Fv (PT7::*BR2scFv*; pBR322 ori, Amp^{R}) | **20** | **BR2ScFvNdeF:** 5'TGATAACATATGCGTGCTGGTCTGCAGT-3' |
| | | **16** | **ScFvXhoR:** 5'-TCATTACTCGAGTCATCATTAGTGGTGGTGGTGGTG GTGTTTGATCTCCAGCTTGGTCC-3' |
| ***br2scfv* with 1x binding loop (NdeI/XhoI)** | pET16b-BRScF v1L (PT7::*BR2scFv-(loop)₁*; pBR322 ori, Amp^{R}) | **21** | **BR2ScFvNdeF:** 5'-TGATAACATATGCGTGCTGGTCTGCAGT-3' |
| | | **17** | **ScFv1LXhoR:** 5'-CCGTTACTCGAGCCGCGCGGGGTGATCTAGGTC CGCGCGGTCGTCGTCGTCATCATTAGTGGTGGTGGTGGTGGTGTT TGATCTCCAGCTTGGTCC-3' |
| **UGD (NdeI/XhoI)** | pET16b-UGD (PT7::*ugd*; pBR322 ori, Amp^{R}) | **22** | **UGDNdeF:** 5'-TGATAACATATGAAAATCACCATTTCCGG-3' |
| | | **23** | **UGDXhoR** 5'-CCGTTACTCGAGTTATTAGTGGTGGTGGTGGTGGTG GTCGCTGCCAAAGAGATCG-3' |
| ***ugd* with 1x** | pET16b-UGD1L | **22** | **UGDNdeF:** 5'-TGATAACATATGAAAATCACCATTTCCGG-3' |
| **binding loop (NdeI/XhoI)** | (PT7::*ugd-(loop)* ₁; pBR322 ori, Amp^{R}) | **24** | **UGD1LXhoR:** 5'-CCGTTACTCGAGCCGCGCGGGGTGATCTAGGTC CGCGCGGTCGTCGTCGTCATCATTAGTGGTGGTGGTGGTGGTGG TCGCTGCCAAAGAGATCG-3' |
| **Adh1p (NdeI/XhoI)** | pET16b-Adh (PT7::*adh1*; pBR322 ori, Amp^{R}) | **25** | **AdhNdeF** 5'-TGATAACATATGTCTATCCCAGAAACTCAAAA-3' |
| | | **26** | **AdhXhoR:** 5'-CCGTTACTCGAGTTATTAGTGGTGGTGGTGGTGGTG TTTAGAAGTGTCAACAACGTATCT-3' |
| ***adh1p* with 1x binding loop (NdeI/XhoI)** | pET16b-Adh1L (PT7::*adh1-(loo p)₁*; pBR322 ori, Amp^{R}) | **25** | **AdhNdeF:** 5'-TGATAACATATGTCTATCCCAGAAACTCAAAA-3' |
| | | **27** | **Adh1LXhoR:** 5'-CCGTTACTCGAGCCGCGCGGGGTGATCTAGGTCC GCGCGGTCGTCGTCGTCATCATTAGTGGTGGTGGTGGTGGTGTTT AGAAGTGTCAACAACGTATCT-3' |
| ***adh1p* with 3x binding loop (NdeI/XhoI)** | pET16b-Adh3L (PT7::*adh1-(loo p)₃*; pBR322 ori, Amp^{R}) | **25** | **AdhNdeF:** 5'-TGATAACATATGTCTATCCCAGAAACTCAAAA-3' |
| | | **28** | **3L-1R:** 5'-AGGTGAGCAACGGACATCCTTCACGGGTGATCTAGGTC GTGAAGGCTCGATCGTCATCATTAGTGGTGGTGGTGGTGGTG-3' |
| | | **29** | **3LXhoR:** 5'-CCGTTACTCGAGTCGTAGAGCGGTGATCTAGGTGCTC TACGGACTGCGTTGCTCGGTGATCTAGGTGAGCAACGGACATCCT TCACG-3' |
| **UbiC (NdeI/XhoI)** | pET16b-UbiC (PT7::*ubiC*; pBR322 ori, Amp^{R}) | **30** | **UbiCNdeF:** 5'-TGATAACATATGCGATTGTTGCGTTTTGTTGC-3' |
| | | **31** | **UbiCXhoR:** 5'-CCGTTACTCGAGTTATTAGTGGTGGTGGTGGTGGT GGTACAACGGTGACGCCGGTA-3' |
| ***ubiC* with 1x binding loop (NdeI/XhoI)** | pET16b-UbiC1L (PT7::*ubiC-(loop )₁*; pBR322 ori, Amp^{R}) | **30** | **UbiCNdeF:** 5'-TGATAACATATGCGATTGTTGCGTTTTTGTTGC-3' |
| | | **32** | **UbiC1LXhoR:** 5'-CCGTTACTCGAGCCGCGCGGGGTGATCTAGGTC CGCGCGGTCGTCGTCGTCATCATTAGTGGTGGTGGTGGTGGTGG TACAACGGTGACGCCGGTA-3' |
| ***ubiC* with 3x binding loop (NdeI/XhoI)** | pET16b-UbiC3L (PT7::*ubiC-(loop* )*₃*; pBR322 ori, Amp^{R}) | **30** | **UbiCNdeF:** 5'-TGATAACATATGCGATTGTTGCGTTTTTGTTGC-3' |
| | | **33** | **3L-1R:** 5'-AGGTGAGCAACGGACATCCTTCACGGGTGATCTAGGT CGTGAAGGCTCGATCGTCATCATTAGTGGTGGTGGTGGTGGTG-3' |
| | | **34** | **3LXhoR:** 5'-CCGTTACTCGAGTCGTAGAGCGGTGATCTAGGTGCTC TACGGACTGCGTTGCTCGGTGATCTAGGTGAGCAACGGACATCCT TCACG-3' |
| **HIV1-Pr (XbaI/XhoI)** | pET16b-HIVpr (PT7::*HIV1Pr*, pBR322 ori, Amp^{R}) | **35** | **HIVPrXbaF:** 5'- ATTCTAAATCTAGATTATTCACTACGCGTTAAGGAG GTACGACATGCACCATCACCACCATCATCCTCAAATCACCCTGTG GC-3' |
| | | **36** | **HIVPrXhoR** 5'-CCGAATTACTCGAGTCATCATTAGAAGTTCAGGGT GCAACCGATCTGGGTCAGCATGTTACGACCGATGATGTTGATCGG GGTCG-3' |
| ***hiv1pr* with 1x binding loop (XbaI/XhoI)** | pET16b-HIVpr1L (PT7:: *HIV1Pr-(loop)₁*; pBR322 ori, Amp^{R}) | **35** | **HIVPrXbaF:** 5'-ATTCTAAATCTAGATTATTCACTACGCGTTAAGGAG GTACGACATGCACCATCACCACCATCATCCTCAAATCACCCTGTG GC-3' |
| | | **37** | **HIVPr1LXhoR:** 5'-CCGTTACTCGAGCCGCGCGGGGTGATCTAGGTC CGCGCGGTCGTCGTCGTCATCATTAGAAGTTCAGGGTGCAACCG-3' |
| ***hiv1pr* with 3x binding loop (NdeI/XhoI)** | pET16b-HIVpr3L (PT7:: *HIV1Pr-(loop)₃*; pBR322 ori, Amp^{R}) | **35** | **HIVPrXbaF:** 5'-ATTCTAAATCTAGATTATTCACTACGCGTTAAGGAG GTACGACATGCACCATCACCACCATCATCCTCAAATCACCCTGTG GC-3' |
| | | **38** | **HIVPrXho3LR:** 5'-CCGAATTACTCGAGCCGCGCGGGGTGATCTAGG TCCGCGCGGTCGTCGTCGTCA TCA TTAGAAGTTCAGGGTGCAACC -3' |
| **Leptin (NdeI/XhoI)** | pET16b-Lep (PT7::*LEP*; pBR322 ori, Amp^{R}) | **39** | **LepNdeF:** 5'-TGATAACATATGCGATTGTTGCGTTTTTGTTGC-3' |
| | | **40** | **LepXhoR:** 5'-CCGTTACTCGAGTTATTAGTGGTGGTGGTGGTGGTG GTACAACGGTGACGCCGGTA-3' |
| ***leptin* with 1x binding loop (NdeI/XhoI)** | pET16b-Lep1L (PT7:: *LEP-(loop)₁*; pBR322 ori, Amp^{R}) | **39** | **LepNdeF:** 5'-TGATAACATATGCGATTGTTGCGTTTTTGTTGC-3' |
| | | **41** | **Lep1LXhoR:** 5'-CCGTTACTCGAGCCGCGCGGGGTGATCTAGGTCC GCGCGGTCGTCGTCGTCATCATTAGTGGTGGTGGTGGTGGTGGT ACAACGGTGACGCCGGTA-3' |
| ***leptin* with 3x binding loop (NdeI/XhoI)** | pET16b-Lep3L (PT7:: *LEP-(loop)₃*; pBR322 ori, Amp^{R}) | **39** | **LepNdeF:** 5'-TGATAACATATGCGATTGTTGCGTTTTTGTTGC-3" |
| | | **42** | **3L-1R:** 5'-AGGTGAGCAACGGACATCCTTCACGGGTGATCTAGGTC GTGAAGGCTCGATCGTCATCATTAGTGGTGGTGGTGGTGGTG-3' |
| | | **43** | **3LXhoR:** 5'-CCGTTACTCGAGTCGTAGAGCGGTGATCTAGGTGCTC TACGGACTGCGTTGCTCGGTGATCTAGGTGAGCAACGGACATCCT TCACG-3' |
| **BMP2 (NdeI/XhoI)** | pET16b-BMP2 (PT7::*BMP2*; pBR322 ori, Amp^{R}) | **44** | **BMP2NdeF** 5'-TGATAACATATGCAAGCCAAACACAAACAG-3' |
| | | **45** | **BMP2XhoR:** 5'-CCGTTACTCGAGTTATTAGTGGTGGTGGTGGTGGT GGCGACACCCACAACCCTC-3' |
| ***bmp2* with 1x binding loop (NdeI/XhoI)** | pET16b-BMP21L (PT7:: *BMP2-(loop)₁*; pBR322 ori, Amp^{R}) | **44** | **BMP2NdeF:** 5'-TGATAACATATGCAAGCCAAACACAAACAG-3' |
| | | **46** | **BMP21LXhoR:** 5'-CCGTTACTCGAGCCGCGCGGGGTGATCTAGGTC CGCGCGGTCGTCGTCGTCATCATTAGTGGTGGTGGTGGTGGTGC GACACCCACAACCCTC-3' |
| ***bmp2* with 3x binding loop (NdeI/XhoI)** | pET16b-BMP23L (PT7:: *BMP2-(loop)₃*; pBR322 ori, Amp^{R}) | **44** | **BMP2NdeF:** 5'-TGATAACATATGCAAGCCAAACACAAACAG-3' |
| | | **47** | **3L-1R:** 5-AGGTGAGCAACGGACATCCTTCACGGGTGATCTAGGTC GTGAAGGCTCGATCGTCATCATTAGTGGTGGTGGTGGTGGTG-3' |
| | | **19** | **3LXhoR:** 5'-CCGTTACTCGAGTCGTAGAGCGGTGATCTAGGTGCTC TACGGACTGCGTTGCTCGGTGATCTAGGTGAGCAACGGACATCCT TCACG-3' |
| **sfGFP (NdeI/EcoRI)** | pET16b-sfGFP (PT7::*sfGFP*; pBR322 ori, Amp^{R}) | **48** | **sfGFPNdeF:** 5'-TGATAACATATGCAAGCCAAACACAAACAG-3' |
| | | **49** | sfGFPEcoR: 5-AGGTCAGAATTCTCATCATTACGTAATACCTGCCG CATTC-3' |
| **C-sfGFP (XbaI/NdeI)** | pET16b-CsfGFP (PT7:: *CsfGFP*; pBR322 ori, Amp^{R}) | **50** | **CsfGFPXbaF:** 5-CCATGATCTAGAAATAATTTTGTTTAACTTTAAGA AGGAGATATACCATGGTGCCCATCCAAAAAGTC-3' |
| | | **51** | **CsfGFPNdeR** 5'- CCGTTACATATGTCATCATTATGTAATCCCAGCA GCATTTAC-3' |
| **N-sfGFP (NdeI/ EcoRI)** | pET16b-CsfGFP -NsfGFP (PT7:: *CsfGFP-NsfGF P*; pBR322 ori, Amp^{R}) | **52** | **sfGFPNdeF** 5'-TGATAACATATGCAAGCCAAACACAAAGAG-3' |
| | | **53** | **sfNGFPEcoR:** 5'-AGGTCAGAATTCTCATCATTATTTTTCGTTCGGAT CTTTAGACA-3' |
| ***nsfgfp* with 3x binding loop (NdeI/EcoRI)** | pET16b-CsfGFP -NsGFP3L (PT7:: *CsfGFP-NsfGF P-(loop)₃*; pBR322 ori, Amp^{R}) | **48** | **sfGFPNdeF:** 5'-TGATAACATATGCAAGCCAAACACAAACAG-3' |
| | | **54** | **sfNGFP3L-1R:** 5'-AGGTGAGCAACGGACATCCTTCACGGGTGATCT AGGTCGTGAAGGCTCGATCGTCATCATTATTTTTCGTTCGGATCTT TAGACA-3' |
| | | **55** | **sfHGFP3LEcoR:** 5'- AGGTCAGAATTCTCGTAGAGCGGTGATCTAGG TGCTCTACGGACTGCGTTGCTCGGTGATCTAGGTGAGCAACGGAC ATCCTTCAC -3' |

### Experimental Example 2. Construction of expression vector encoding fusion protein of chaperone and RNA-binding domain or target recombinant protein for CRAS s^{y}stem

For a chaperone-recruiting mRNA scaffold (CRAS) system, an expression vector encoding a fusion protein of a chaperone and an RNA-binding domain or a target recombinant protein was constructed.

First, in order to prepare a medium-copy number vector, based on a pACYCDuet-1 vector having two strong T7 promoters (Novagen, Madison, WI, USA), p15A which is a low-copy number origin of replication was replaced by pBR322 which is a medium-copy number origin of replication to prepare a pAMT7 vector.

Chaperone genes, *dnaJ* (SEQ ID NO: 74) and *dnaK* (SEQ ID NO: 75), were amplified by PCR, based on genomic DNA of *E.coli* BL21(DE3). For high expression of *dnaJ* and *dnaJ-KH,* a synthetic ribosome-binding site was designed by using a ribosome-binding site (RBS) calculator, and incorporated into the 5'-terminus of DnaJNcoF primer.

To prepare the fusion protein of the chaperone and the RNA-binding domain, a gene (SEQ ID NO: 76) of Nova-1 KH3 RNA-binding domain (KH) having a sequence optimized for expression in *E.coli* was synthesized and bound downstream of *dnaJ* or *dnaJK* by recombinant PCR.

A gene encoding the target recombinant protein was amplified by PCR, based on various origins: *scfu* and *br2-scfu* were amplified based on pBR2ScFv (PloS one, 2013, 8, e66084), *ugd* and *ubiC* were amplified based on the genomic DNA of *E.coli* BL21(DE3), and *adh1p* was amplified based on the genomic DNA of *S. cerevisae.* HIV1-Pr gene was prepared by recombinant PCR (Protein expression and purification, 2015, 116, 59-65). *tliA* was amplified based on pTliA (Acs Catal, 2015, 5, 5016-5025). For green fluorescent protein (GFP) complementation assay, superfolder green fluorescent protein (sfGFP) (Bioneer, Daejeon, Korea) was used as a template to amplify the N-terminus and the C-terminus of sfGFP. For high expression of sfGFP, synthetic RBS and the N-terminus of sfGFP were designed by using an RBS calculator and incorporated into the 5'-terminus of sfGFPXbaF primer. The used primers are shown in Table 1.

For purification, 6×His-tag was added at the N-terminus of a forward primer for HIV1-Pr and at the C-terminus of a reverse primer for the target recombinant protein. For high expression of HIV1-Pr, synthetic RBS was designed by using the RBS calculator, and included at the 5'-terminus of HIVPrXbaF primer. PCR products and expression vectors corresponding thereto were digested with restriction enzymes in Table 1. Then, the chaperone was ligated to pAMT7 and the recombinant protein was ligated to pET16b.

### Experimental Example 3. Preparation of RNA scaffold for CRAS system

For the chaperone-recruiting mRNA scaffold (CRAS) system, an RNA scaffold was prepared. In detail, a sequence of a binding loop for KH domain used in the RNA scaffold system was designed by RNA Designer and mFold (Journal of molecular biology, 2004, 336, 607-624; Science 1989, 244, 48-52). A sequence constraint is as follows;

5'-NNNNNNNNACCTAGATCACC(SEQ ID NO: 77)NNNNNNNN-3', wherein N represents an arbitrary nucleotide of 8 bp for a stem structure, and a sequence underlined represents a loop structure including a binding sequence for the KH RNA-binding domain.

In the case of an RNA scaffold system having a plurality of binding loops, a stem-loop structure was designed by a similar approach to add a 5-nt spacer between respective stem-loop structures. A stem-loop structure for the target protein gene was prepared by PCR using a reverse primer having a sequence of the stem-loop structure in 3'-UTR.

### Experimental Example 4. Preparation of CLEX system

CLEX system of DnaJ chaperone and recombinant protein (ScFv, UbiC, Leptin, HIV1-Pr, BMP2, or TliA) was prepared in two different arrangements in which DnaJ was placed in the first or second cistron. A 12-nt region (5'-GAGGAGGTGGAA-3', SEQ ID NO: 79) encoding an amino acid sequence of EEVE (EEVE, SEQ ID NO: 78) and including a Shine-Dalgarno (SD) sequence (underlined) was introduced at the C-terminus of the gene in the first cistron in order to improve translation initiation of the gene in the second cistron. To secure translational coupling and to promote interaction between DnaJ and the recombinant protein, the stop codon of the first cistron and the start codon of the second cistron were overlapped through 1 nt (5'-TAATG-3'). To prepare the CLEX system, the DnaJ chaperone and the recombinant protein were assembled by recombinant PCR. Primer sequences are shown in Table 2. Each PCR product and pET16b were digested by restriction enzyme described in Table 2, and ligated to the expression vector.

**[Table 2]**

| **Proteins** | **Plasmid** | SEQ ID NO. | **Primers (5' to 3')** |
|---|---|---|---|
| **DnaJ/ScFv (NdeI/XhoI)** | pET16b-DnaJSc Fv (PT7::*dnaJ-ScF v*; pBR322 ori, Amp^{R}) | **56** | **DnaJMdeF**:5-TGATAACATATGGCTAAGCAAGATTATTACG-3' |
| | | **57** | **ScFvFpol**:5'-GTTTTTTGACGACCTGACCCGCGAGGAGGTGG AATAATGCAGGTCCAACTGCAGC-3' |
| | | **16** | **ScFvXhoR**:5'-TCATTACTCGAGTCATCATTAGTGGTGGTGGT GGTGGTGTTTGATCTCCAGCTTGGTCC-3' |
| **ScFv/DnaJ (NdeI/XhoI)** | pET16b-ScFvDn aJ (PT7::*ScFv-dna J*; pBR322 ori, Amp^{R}) | **15** | **ScFvNdeF**: 5'-TGATAACATATGCAGGTCCAACTGCAGC-3' |
| | | **58** | **DnaJFpol**:5'-CACCACCACCACCACCACGAGGAGGTGGAATA ATGGCTAAGCAAGATTATTACG-3' |
| | | **2** | **DnaJXhoR**: 5'-ATTTCACTCGAGTTATTAGCGGGTCAGGTCGTCAAA-3' |
| **DnaJ/UbiC (NdeI/XhoI)** | pET16b-DnaJU biC (PT7::*dnaJ-ubiC* ; pBR322 ori, Amp^{R}) | **56** | **DnaJNdeF**:5-TGATAACATATGGCTAAGCAAGATTATTACG-3' |
| | | **59** | **UbiCFpol**:5-GTTTTTTGACGACCTGACCCGCGAGGAGGTGG AATAATGCGATTGTTGCGTTTTTGTTGC-3' |
| | | **31** | **UbiCXhoR**:5'-CCGTTACTCGAGTTATTAGTGGTGGTGGTGGT GGTGGTACAACGGTGACGCCGGTA-3' |
| **UbiC/DnaJ (NdeI/XhoI)** | pET16b-UbiCDn aJ (PT7::*ubiC-dnaJ* ; pBR322 ori, Amp^{R}) | **30** | **UbiCNdeF:** 5-TGATAACATATGCGATTGTTGCGTTTTTGTTGC-3' |
| | | **58** | **DnaJFpol**:5'-CACCACCACCACCACCACGAGGAGGTGGAATA ATGGCTAAGCAAGATTATTACG-3' |
| | | **2** | **DnaJXhoR:** 5'-ATTTCACTCGAGTTATTAGCGGGTCAGGTCGTCAAA-3' |
| **DnaJ/HIV1-Pr (NdeI/XhoI)** | pET16b-DnaJHI V1Pr (PT7::*dnaJ-HIVI Pr*, pBR322 ori, Amp^{R}) | **56** | **DnaJNdeF**:5'-TGATAACATATGGCTAAGGAAGATTATTACG-3' |
| | | **60** | **HIVPrFpol:** 5'-GTTTTTTGACGACCTGACCCGCGAGGAGGTGGAATAATG CACCATCACCACCATCATCCTCAAATCACCCTGTGGC-3' |
| | | **36** | **HIVPrXhoR**:5'-CCGAATTACTCGAGTCATCATTAGAAGTTCAG GGTGCAACCGATCTGGGTCAGCATGTTACGACCGATGATG TTGATCGGGGTCG-3' |
| **HIV1-Pr/DnaJ (XbaI/XhoI)** | pET16b-HIV1Pr DnaJ (PT7:: *HIVIPr-dnaJ*; pBR322 ori, Amp^{R}) | **35** | **HIVPrXbaF**:5'-ATTCTAAATCTAGATTATTCACTACGCGTTAAG GAGGTACGACATGCACCATCACCACCATCATCCTCAAATCA CCCTGTGGC-3' |
| | | **61** | **DnaJFpoIHIVPr**:5'-CGGTTGCACCCTGAACTTCGAGGAGGTG GAATAATGGCTAAGCAAGATTATTACG-3' |
| | | **2** | **DnaJXhoR:** 5'-ATTTCACTCGAGTTATTAGCGGGTCAGGTCGT CAAA-3' |
| **DnaJ/Leptin (NdeI/XhoI)** | pET16b-DnaJLe p (PT7::*dnaJ-LEP;* pBR322 ori, Amp^{R}) | **56** | **DnaJNdeF**:5'-TGATAACATATGGCTAAGCAAGATTATTACG-3' |
| | | **62** | **LepFpol**: 5'-GTTTTTTGACGACCTGACCCGCGAGGAGGTGGAATAATG CGATTGTTGCGTTTTTGTTGC-3' |
| | | **40** | **LepXhoR**:5'-CCGTTACTCGAGTTATTAGTGGTGGTGGTGGT GGTGGTACAACGGTGACGCCGGTA-3' |
| **Leptin/DnaJ (NdeI/XhoI)** | pET16b-LepDna J (PT7:: *LEP-dnaJ*; pBR322 ori, Amp^{R}) | **39** | **LepNdeF**: 5'-TGATAACATATGCGATTGTTGCGTTTTTGTTGC-3' |
| | | **58** | **DnaJFpol**-5'-CACCACCACCACCACCACGAGGAGGTGGAATA ATGGCTAAGCAAGATTATTACG-3' |
| | | **2** | **DnaJXhoR**: 5'-ATTTCACTCGAGTTATTAGCGGGTCAGGTCGTCAAA-3' |
| **DnaJ/BMP2 (NdeI/XhoI)** | pET16b-DnaJB MP2 (PT7::*dhaJ-BMP* 2; pBR322 ori, Amp^{R}) | **56** | **DnaJNdeF:** 5'-TGATAACATATGGCTAAGCAAGATTATTACG-3' |
| | | **63** | **BMP2FPol**:5'-GTTTTTTGACGACCTGACCCGCGAGGAGGTG GAATAATGCAAGCCAAACACAAACAG-3' |
| | | **45** | **BMP2XhoR**:5'-CCGTTACTCGAGTTATTAGTGGTGGTGGTGG TGGTGGCGACACCCACAACCCTC-3' |
| **BMP2/DnaJ (NdeI/XhoI)** | pET16b-BMP2DnaJ (PT7:: *BMP2 -dnaJ;* pBR322 ori, Amp^{R}) | **44** | **BMP2NdeF:** 5'-TGATAACATATGCAAGCCAAACACAAACAG-3' |
| | | **58** | **DnaJFpol**:5'-CACCACCACCACCACCACGAGGAGGTGGAATA ATGGCTAAGCAAGATTATTACG-3' |
| | | **2** | **DnaJXhoR**: 5'-ATTTCACTCGAGTTATTAGCGGGTCAGGTCGTCAAA-3' |
| **DnaJ/Adh1p (NdeI/XhoI)** | pET16b-DnaJAd h (PT7::*dnaJ-adh1;* pBR322 ori, Amp^{R}) | **56** | **DnaJNdeF**: 5'-TGATAACATATGGCTAAGCAAGATTATTACG-3' |
| | | **64** | **AdhFPol:**5'-GTTTTTTGACGACCTGACCCGCGAGGAGGTGG AATAATGTCTATCCCAGAAACTCAAAA-3' |
| | | **26** | **AdhXhoR**:5'-CCGTTACTCGAGTTA TT AGTGGTG GTGGTGGT GGTGTTTAGAAGTGTCAACAACGTATCT-3' |
| **Adh1p/DnaJ (NdeI/XhoI)** | pET16b-AdhDnaJ (PT7:: *adh1-dnaJ*; pBR322 ori, Amp^{R}) | **25** | **AdhNdeF:** 5'-TGATAACATATGTCTATCCCAGAAACTCAAAA-3' |
| | | **58** | **DnaJFpol**:5'-CACCACCACCACCACCACGAGGAGGTGGAATA ATGGCTAAGCAAGATTATTACG-3' |
| | | **2** | **DnaJXhoR**: 5'-ATTTCACTCGAGTTATTAGCGGGTCAGGTCGTCAAA-3' |
| **TliA (NdeI/EcoRI)** | pET16b-TliA (PT7:: *tliA;* pBR322 ori, Amp^{R}) | **65** | **TliANdeF**:5'-TGATAACATATGCATCATCATCATCATCATCATC ATCACAGCA-3' |
| | | **66** | **TliAEcoR**:5'-CTGAGAATTCTCATCATTAACTGATCAGCACAC CCTCGCTCC-3' |
| **DnaJ/TliA (NdeI/EcoRI)** | pET16b-DnaJTli A (PT7::*dnaJ-tliA*; pBR322 ori, Amp^{R}) | **56** | **DnaJNdeF**: 5'-TGATAACATATGGCTAAGCAAGATTATTACG-3' |
| | | **67** | **TliAFPol**:5'-GTTTTTTGACGACCTGACCCGCGAGGAGGTGG AATAATG CATCATCATCATCATCATCATCATCACAGCA-3' |
| | | **66** | **TliAEcoR**:5'-CTGAGAATTCTCATCATTAACTGATCAGCACAC CCTCGCTCC-3' |
| **TliA/DnaJ (NdeI/EcoRI)** | pET16b-TliADnaJ (PT7:: *tliA-dnaJ*; pBR322 ori, Amp^{R}) | **65** | **TliANdeF**:5'-TGATAACATATGCATCATCATCATCATCATCATC ATCACAGCA-3' |
| | | **68** | **DnaJTIiAFpol**:5'-GGAGCGAGGGTGTGCTGATCAGTGAGGA GGTGGAATAATGGCTAAGCAAGATTATTACG-3' |
| | | **69** | **DnaJEcoR**: 5'-CTGAGAATTCTTATTAGCGGGTCAGGTCGTCAA A-3' |
| **TliA1 (NdeI/EcoRI)** | pET16b-TliA1 (PT7:: *tliA* (*aa 1-300*); pBR322 ori, Amp^{R}) | **65** | **TliANdeF**:5'-TGATAACATATGCATCATCATCATCATCATCATC ATCACAGCA-3' |
| | | **70** | **TliA1EcoR**:5'-CCGTTAGAATTCTCATCATTAGTCGGTGGTCG ACTCGTG-3' |
| **DnaJ/TliA1 (NdeI/EcoRI)** | pET16b-DnaJTli A1 (PT7::*dnaJ-tliA* (*aa 1-300*); pBR322 ori, Amp^{R}) | **56** | **DnaJNdeF**:5'-TGATAACATATGGCTAAGCAAGATTATTACG-3' |
| | | **71** | **TliA1FPol**:5'-AGTTTTTTGACGACCTGACCCGCACCGGAGGT ACATAATGCATCATCATCATCATCATCATCATCACAGCA-3' |
| | | **72** | **TliA1EcoR**:5'-CCGTTAGAATTCTCATCATTAGTCGGTGGTCG ACTCGTG-3' |
| **TliA2 (NdeI/EcoRI)** | pET16b-TliA2 (PT7::*tliA (aa 301-493);* pBR322 ori, Amp^{R}) | **73** | **TliA2NdeF**:5-TGATAACATATGAACATCGTCAGCTTCAACG-3' |
| | | **66** | **TliAEcoR**:5'-CTGAGAATTCTCATCATTAACTGATCAGCACAC CCTCGCTCC-3' |

### Experimental Example 5. Method of measuring protein solubilization

*E.coli* BL2(DE3) was co-transformed with the plasmid of the chaperone and the plasmid of the target recombinant protein. Thereafter, the *E.coli* was inoculated in 3 ml of LB (Lysogenic Broth) medium (10 g/l tryptone, 5 g/l yeast extract, and 10 g/l sodium chloride) supplemented with ampicillin and chloramphenicol at a final concentration of 50 µg/ml and 25 µg/ml, respectively and cultured in a rotary shaker (200 rpm) at 37°C overnight. Then, 1 ml of the culture was inoculated in 100 ml of LB medium supplemented with ampicillin and chloramphenicol at a final concentration of 50 µg/ml and 25 µg/ml, respectively and then cultured at 200 rpm and 37°C. When OD₆₀₀ reached 0.5-0.6, 0.5 mM isopropyl β-d-1-thiogalactopyranoside (IPTG) was added to induce expression of the chaperone and the target protein, and then, further cultured for 4 hours (37°C, 200 rpm). Thereafter, when OD₆₀₀ value reached 2, 3 ml of cell culture (including about 1.6 × 10⁹ cells) was centrifuged and resuspended in 500 µl of 10 mM Tris-EDTA (TE) buffer (pH 7.6), followed by sonication. Soluble and insoluble fractions were separated by centrifugation (21,600×g, 15 min, 4°C). An insoluble pellet was washed with 1% Triton X-100 twice, and resuspended in 10 mM TE buffer (pH 7.6) and used as an insoluble fraction. Solubility of the target recombinant protein was measured by dodecyl sodium sulfate polyacrylamide gel electrophoresis (SDS-PAGE). Band intensity of the whole cell lysates and the soluble fractions of the target recombinant proteins stained with Coomassie Blue in the SDS-PAGE gel were compared by using an ImageJ software to calculate relative percentages of the soluble target recombinant proteins.

### Experimental Example 6. Method of purifying protein

*E.coli* BL21(DE3) having pAMT7-DnaJ-KH was cultured in an LB medium supplemented with chloramphenicol at a final concentration of 25 µg/ml. The *E.coli* was cultured in a rotary shaker (200 rpm) at 37°C overnight. Then, 1 ml of the culture was inoculated in 100 ml of LB medium supplemented with chloramphenicol at a final concentration of 25 µg/ml, and cultured at 200 rpm at 37°C. When OD₆₀₀ reached 0.6, 0.5 mM IPTG was added to induce expression of the DnaJ-KH protein, and then, further cultured for 4 hours (37°C, 200 rpm). Thereafter, 20 ml of the cells were harvested by centrifugation, and resuspended in 1× Native IMAC lysis buffer (Biorad, Hercules, CA, USA), followed by sonication. Transparent cell lysate was further centrifuged (21,600×g, 15 min, 4°C). DnaJ-KH was purified from a soluble fraction by a Native IMAC method of an automated Profinia™ protein purification system (Biorad, Hercules, CA, USA). Thereafter, the purified DnaJ-KH was dialyzed against phosphate-buffered saline (PBS) at pH 7.4 and 25 mM Tris-HCl buffer (pH 8.8) containing 5 mM DTT, 100 mM NaCl, and 10% glycerol.

### Experimental Example 7. GFP complementation assay

Correct folding of the recombinant protein in the CRAS system was evaluated by a known GFP complementation assay (Nature methods, 2012, 9, 671-675) with slight modification. *E.coli* BL21(DE3) having pAMT7-DnaJK-KH and pET16b-sfGFP/pET16b-CsfGFP-NsfGFP/pET16b-CsfGFP-NsfGFP3L was inoculated in an LB medium supplemented with ampicillin and chloramphenicol at a final concentration of 50 µg/ml and 25 µg/ml, respectively and cultured in a rotary shaker (200 rpm) at 37°C overnight. Then, 1 ml of the culture was inoculated in 100 ml of LB medium supplemented with ampicillin and chloramphenicol at a final concentration of 50 µg/ml and 25 µg/ml, respectively, and cultured at 200 rpm at 37°C. When OD₆₀₀ reached 0.6, 0.5 mM IPTG was added to induce expression of DnaJ-KH and the target protein, and then, further cultured for 4 hours (37°C, 200 rpm). Thereafter, 1 ml of the cells were harvested by centrifugation, and resuspended in 500 µl of PBS (pH 7.4) and diluted until OD₆₀₀ reached 1. Thereafter, the cells were loaded in a 96-well black plate (SPL Life Sciences, Pocheon, Korea). Tecan Infinite F200 PRO instrument (Tecan Group Ltd., Mannedorf, Switzerland) was used to measure fluorescence intensity of each well (λₑₓₑ = 488 nm / λₑₘ = 530 nm).

### Experimental Example 8. Electric Mobility Shift Assay (EMSA)

mRNA of anti p21ras-ScFvd having no loop, one loop, or three loops was prepared by using a HiScribe™ T7 High Yield RNA Synthesis Kit (New England Biolab, Beverly, MA). PBS (pH 7.4) having 0.1 nM mRNA and 200 µM of purified DnaJ-KH were mixed. Thereafter, the mixture was incubated at 25°C for 30 minutes, and analyzed by electrophoresis on 2% agarose gel in Tris-boric acid-EDTA buffer. Electrophoresis was performed at constant 50 V for 20 minutes. Thereafter, the gel was visualized by using a Gel-Doc gel documentation system (Bio-Rad, Hercules, CA).

### Experimental Example 9. Method of measuring leptin activity

*E.coli* BL21(DE3) having pET16b-DnaJLep or pET16b-Lep was cultured in an LB medium supplemented with ampicillin at a final concentration of 50 µg/ml in a rotary shaker (200 rpm) at 37°C overnight. Then, 1 ml of the culture was inoculated in 100 ml of LB medium supplemented with ampicillin at a final concentration of 50 µg/ml, and cultured at 200 rpm at 37°C. When OD₆₀₀ reached 0.6, 0.5 mM IPTG was added and then, further cultured for 4 hours (37°C, 200 rpm) to induce expression of DnaJ and leptin. Thereafter, 20 ml of the cells were harvested by centrifugation, and resuspended in 1× PBS (pH 7.4), followed by sonication. Transparent cell lysate was further centrifuged (21,600×g, 15 min, 4°C). Activity of the purified leptin was analyzed by ELISA. 40 µg of a soluble fraction of the transparent cell lysate (8 mg/ml) was loaded in a 96-well plate (Thermo Fisher Scientific, Waltham, MA, USA) with carbonate- bicarbonate buffer at 37°C for 1 hour. Thereafter, the plate was washed with 1× PBS-T (1× PBS containing 0.5% Tween-20) three times, and then each well was blocked with bovine serum albumin (Invitrogen, Carlsbad, CA, USA) for 1 hour. Thereafter, an anti-leptin antibody (Abcam, Cambridge, UK) was added to each well, and incubated at room temperature for 1 hour. After washing the plate, the plate was incubated with a horseradish peroxide-conjugated anti-rabbit monoclonal antibody (Cell Signaling Technology, Beverly, MA, USA) at room temperature for 1 hour. Thereafter, the plate was washed with 1× PBS-T four times, and to initiate peroxidase reaction, 100 µl of TMB (3,3',5,5'-Tetramethylbenzidine) peroxidase substrate (BD, Franklin Lakes, NJ, USA) was added. To stop the reaction, 50 µl of 2 M H₂SO₄ was added to each well. An ELISA reader (Infinite M200 PRO; Tecan Group Ltd., Mannedorf, Switzerland) was used to measure absorbance of each well at 450 nm wavelength.

### Example 1. Result of applying various insoluble proteins to CRAS system

DnaJ, which is an essential element for a molecular chaperone system in bacteria, interacts with a translated or misfolded protein and promotes interaction between foldase chaperone such as DnaK or GroESL and a protein in order to produce a correctly folded protein. For rapid folding immediately after translation, DnaJ was placed at the translation termination site of the target protein to increase spatial proximity between DnaJ and the target protein. For anchoring, human Nova-1 protein-derived sequence-specific RNA-binding domain (KH) was bound with DnaJ (FIG. 1A). Interaction between DnaJ-KH (complex of DnaJ and KH) and 3'UTR hairpin loop was examined by a gel retardation assay. In order to increase solubility of insoluble recombinant proteins such as anti-Ras ScFv (single chain variable fragment), a complex of ScFv and an antimicrobial peptide BR2 (BR2-ScFv), UDP-6-glucose dehydrogenase (UGD), UbiC (pyruvate chorismate lyase), BMP2 (bone morphogenetic protein 2), leptin, Adhip (alcohol dehydrogenase 1p), HIV-1 protease (HIV1-Pr) of *E.coli,* etc., these proteins were applied to the CRAS (chaperone recruiting mRNA scaffold) system.

As a result, when the CRAS system was applied, solubility of ScFv, BR2-ScFv, and UGD was greatly increased, as compared with co-expression of the protein and DnaJ-KH without spatial restriction (without binding loops) or single expression of the protein (FIG. 1B).

### Example 2. Result of solubilization according to number of 3'UTR hairpin loop and distance between stop codon and first loop in CRAS system

The CRAS system did not increase solubility of Adhip, HIV1-Pr, UbiC, Leptin, and BMP2 (FIG. 1B). To solve this problem, the number of 3'UTR hairpin loop was increased. First, a number of 3'UTR hairpin loops were designed and verified through computer simulation to create desired structures (FIG. 1C).

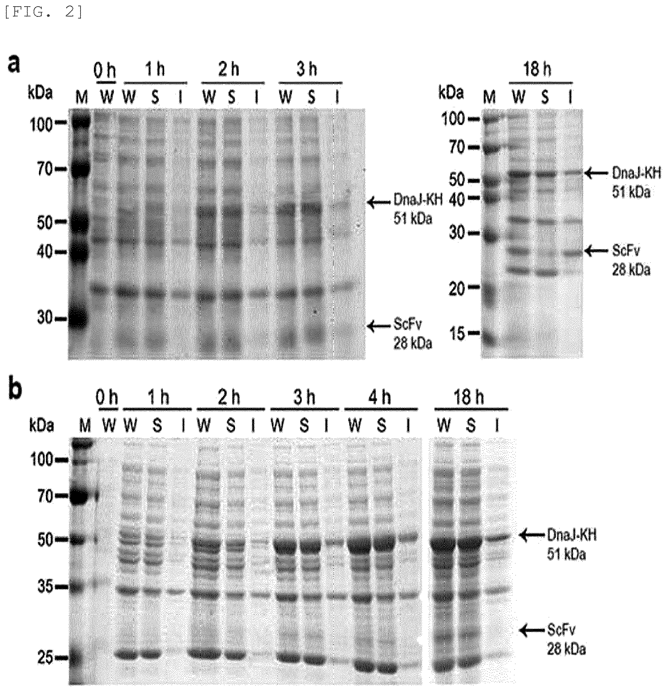

Up to three of 3'UTR hairpin loops showed no significant effect on solubility increase of the protein. However, in this case, a percentage of the soluble fraction of ScFv was maintained constant for 18 hours of expression, whereas the solubility of the protein having one 3'UTR hairpin loop was gradually reduced (FIG. 2), which is very important in increasing the protein production yield.

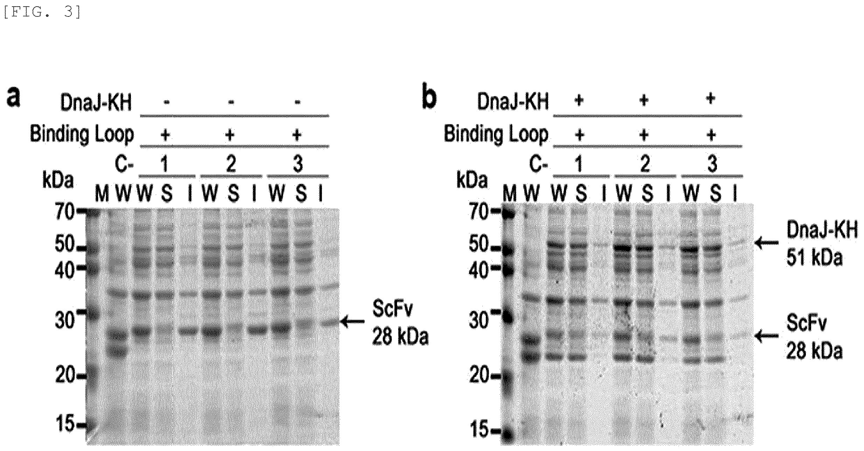

Further, a space of 30 nt or less between the stop codon and the first loop structure had no influence on solubility increase of ScFv (FIG. 3), indicating that the system has flexibility about the distance between the stop codon and DnaJ.

### Example 3. Result of applying CRAS system in ΔdnaJ strain and ΔdnaK strain

DnaK chaperone system is naturally present in *E.coli,* and the system of the present invention anchors the DnaJ chaperone to a translation site, and therefore, *dnaJ* or *dnaK* was deleted in the genome of *E.coli,* and then whether ScFv solubility was increased or not was analyzed by using CRAS system. Since only DnaJ targeting mRNA is able to efficiently interact with ScFv, and the function of the CRAS system may be reduced in *ΔdnaK* strain due to lack of foldase chaperone, it was predicted that the same solubility enhancement efficiency would be observed in the *ΔdnaJ* strain. The *ΔdnaJ* strain was as predicted, but the *ΔdnaK* strain showed slightly increased solubility of ScFV (FIG. 4), suggesting that the CRAS system increased foldase activity of DnaJ so that DnaJ alone functions as the chaperone to increase solubility of the recombinant protein.

### Example 4. Result of applying DnaJ-DnaK-KH complex to CRAS system

It was intended to increase efficiency of the CRAS system by a method of restricting the spatial extent of the chaperone proteins and by a method of increasing the turnover number of each chaperone. To this end, a chimeric chaperone DnaJ-DnaK-KH (DnaJK-KH), which is a complex of DnaJ, DnaK, and KH, was prepared and applied to the CRAS system.

As a result, it was found that solubility of the insoluble proteins was very greatly increased, and up to 90% of the expressed proteins had soluble forms (FIG. 1D). Further, as the numbers of DnaJK-KH and KH hairpin loops were increased, solubility of the target proteins was rapidly increased after protein expression, as compared with DnaJ-KH (FIG. id), suggesting that the number of chaperones adjacent to the translation process is a limiting factor in the folding reactions.

### Example 5. Result of GFP complementation assay in CRAS system

To examine functional folding of the target protein *in vivo*, a GFP complementation assay was designed by splitting superfolder green fluorescent protein (sfGFP) into N-terminus (N-sfGFP) and C-terminus (C-sfGFP) (FIG. 1E). Since N-sfGFP has low solubility, it was selected as a target protein to investigate function of the CRAS system. A fluorescence level represents a level of solubilized functional N-sfGFP. When DnaJK-KH and N-sfGFP interacting therewith are coexpressed with C-sfGFP, fluorescence intensity of cells coexpressing the two fragments was increased twice. In contrast, when three loops were introduced into 3'UTR of N-sfGFP interacting with DnaJ-KH, fluorescence intensity was increased about 8 times, which corresponds to 90% of the fluorescence intensity emitted from the full length protein of sfGFP, indicating that the CRAS system may increase not only solubility but also expression of functional active proteins (FIG. 1F).

### Example 6:

### Example 6-1. Result of applying CLEX system

To demonstrate importance of spatial restriction to the chaperone and the substrate without limiting the number of chaperone adjacent to the translational machinery, a CLEX (chaperone-substrate co-localized expression) system which is a translationally coupled two-cistron expression system capable of solubilizing very insoluble proteins was prepared (FIG. 5A). The overlapping (5'-TAATG-3') of the stop codon and the start codon of the first and second cistrons prevents a transcript from exiting the ribosomal complex and allows translation of the second cistron. Further, the CLEX system was designed such that DnaJ was linked to the target protein-encoding gene of the first or second cistron, and the first cistron has 5'-GAGGAGGTGGAA-3' sequence (SEQ ID NO: 79) (underlined sequence: RBS or Shine-Dalgarno sequence) including the ribosome-binding site (RBS) for the second cistron. With regard to an integration sequence between two cistrons, two overlapping cistrons, two cistrons in tandem, and two cistrons having a space of n bp therebetween were designed (FIG. 5B).

As a result, the CLEX system having the overlapping stop-start codons was very effective in solubilizing very insoluble proteins including BMP2, which are not solubilized by the CRAS system. On average, 60% to 90% of the recombinant proteins were obtained as soluble forms (FIG. 5C). Particularly, correct folding of leptin was confirmed by using a structure-specific antibody according to the method of Experimental Example 9 (FIG. 5D).

Further, the role of DnaJ was determined by the order of the two cistrons, which generates a difference in the expression level and reaction time (FIG. 5C). When the chaperone was expressed from the first cistron, a larger number of soluble fractions were identified. This is because the available chaperone may be prepared prior to translation of the second cistron. Further, the arrangement of DnaJ-BMP2 showed longer protein expression (up to 18 hours after induction) than its reverse arrangement (FIG. 5E), indicating that the timely interaction between the chaperone and the protein is more important than the amount of constituents involved in the chaperone reaction.

Further, it was confirmed that the protein solubility may be increased by increasing a sequence between the genes to 10 nt (FIG. 5F). The nucleotides added between the two genes changed not only a stoichiometric ratio between the two proteins but also spatial proximity between DnaJ and the target recombinant protein, thereby changing the secondary structure of the transcript and affinity for translation initiation of the second cistron.

### Example 6-2. Result of applying CLEX system in ΔdnaK strain

In order to identify the role of DnaK in solubilization of the recombinant protein by the CLEX system, *ΔdnaK* strain was used (FIG. 6).

As a result, it was confirmed that DnaJ was able to sufficiently solubilize BMP2 protein even in the absence of DnaK in the CLEX system, indicating that DnaJ has foldase activity independently of DnaK or other chaperone system is involved in the protein folding in *E.coli.* Accordingly, it can be seen that the CLEX system using the single chaperone DnaJ has very high efficiency.

### Example 7. Result of applying TliA 1 to CLEX system

Although various recombinant proteins were successfully solubilized, solubility of a high molecular weight protein such as TliA (52 kDa lipase) was not highly increased. This is because high molecular weight proteins tend to have many domains that are likely to be misfolded, and the system of the present invention is insufficient to promote the interaction between the chaperone and a number of substrates before irreversible misfolding. To examine this, *tliA* gene was cut into two fragments (*tliA1*, *tliA2*), and then applied to the system of the present invention.

As a result, TliAl was very insoluble whereas TliA2 was soluble (FIGS. 3A and 3B). In the CLEX system where *tilA1* was expressed from the second citron, about 60% of TliAl was solubilized. In contrast, when TliAl and DnaJ were simply coexpressed, 25% of TliAl was solubilized (FIG. 3B), suggesting that the intrinsic properties of TliA, such as protein length and domain arrangement, are the main causes of insolubility, and the solubility of high molecular weight proteins may be increased through the system of the present invention which is capable of sufficiently exposing an area which is likely to be misfolded to the chaperone.

The CRAS system places the chaperone at the 3'UTR of mRNA whereas the CLEX system produces a new chaperone molecule together with the target protein to promote a rapid interaction therebetween. Considering solubilization activity, the CLEX system is superior to the CRAS system. However, the CLEX system is limited in solubilizing a number of proteins at the same time, because *dnaJ* must be cloned into two cistron systems for each recombinant protein. In contrast, the CRAS system having three loops and DnaJK-KH may improve the solubility of many proteins expressed in the cells by simply introducing the 3'UTR KH-binding hairpin sequence into each mRNA. Thus, the CRAS system may be used to increase soluble fractions of functional enzymes involved in the metabolic pathway, and the CLEX system is more suitable for the production of one kind of recombinant protein, such as therapeutic peptides, enzymes, or peptides.

Based on the above description, it will be understood by those skilled in the art that the present invention may be implemented in a different specific form without changing the technical spirit or essential characteristics thereof. Therefore, it should be understood that the above embodiment is not limitative, but illustrative in all aspects. The scope of the invention is defined by the appended claims rather than by the description preceding them, and therefore all changes and modifications that fall within metes and bounds of the claims, or equivalents of such metes and bounds are therefore intended to be embraced by the claims.

### Effect of the invention

A CRAS system of the present invention including 3'UTR hairpin sequence-introduced mRNA of a target protein and a fusion protein of a chaperone and an RNA-binding domain may improve solubilization of various target proteins. Further, a CLEX system of the present invention including two cistrons may improve solubilization of various target proteins.

## Claims

1. A composition for use in solubilizing a target protein, the composition comprising an expression cassette including a promoter and a gene encoding a fusion protein of a chaperone and an RNA-binding domain, or an expression vector including the expression cassette, and optionally,
wherein the RNA-binding domain is K Homology (KH) domain, bacteriophage MS2 coat protein, bacteriophage PP7 coat protein, sterile alpha motif (SAM), or RNA recognition motif (RRM).

2. The composition of claim 1, wherein the chaperone is one or more selected from the group consisting of Hsp40, Hsp60, Hsp70, Hsp90, Hsp100, and Hsp104.

3. The composition according to any one of the preceding claims, wherein the chaperone is one or more selected from the group consisting of DnaJ, Dnak, GroEL, GroES, HtpG, ClpA, ClpX, ClpP, and GrpE.

4. The composition according to any one of the preceding claims, wherein the chaperone of the fusion protein is fused to the N-terminus or C-terminus of the RNA-binding domain.

5. The composition according to any one of the preceding claims, wherein the chaperone of the fusion protein is fused to the RNA-binding domain via a linker.

6. The composition according to any one of the preceding claims, further comprising an expression cassette including a promoter, a gene encoding a target protein, and a gene of a hairpin structure at the 3'-terminus thereof; or an expression vector including the expression cassette, and optionally, wherein the gene of the hairpin structure
(a) has one or more of the gene of the hairpin structure; or
(b) includes a sequence transcribing an RNA site to which the RNA-binding domain linked to the chaperone binds.

7. The composition according to any one of the preceding claims, wherein the expression cassette or the expression vector further comprises a ribosome-binding site (RBS).

8. The composition according to any one of the preceding claims, wherein the expression cassette or the expression vector further comprises a spacer gene between the gene encoding the target protein and the gene of the hairpin structure.

9. The composition according to any one of the preceding claims, wherein the hairpin structure comprises a spacer gene between the hairpin structure and the hairpin structure.

10. A transformant comprising an expression cassette including a promoter and a gene encoding a fusion protein of a chaperone and an RNA-binding domain, or an expression vector including the expression cassette, and optionally,
wherein the transformant is selected from a plant cell, a bacterial cell, a fungal cell, a yeast cell, and an animal cell.

11. The transformant of claim 10, further comprising an expression cassette including a promoter, a gene encoding a target protein, and a gene of a hairpin structure at the 3'-terminus thereof; or an expression vector including the expression cassette.

12. An expression cassette comprising a promoter and a gene encoding a fusion protein of a chaperone and an RNA-binding domain.

13. A kit for use in producing a target protein, the kit comprising the composition according to any one of the preceding claims, or the transformant according to any one of the preceding claims.

14. A method for producing a target protein, the method comprising the steps of:
(i) preparing a transformant including (a) an expression cassette including a promoter and a gene encoding a fusion protein of a chaperone and an RNA-binding domain, or an expression vector including the expression cassette and (b) an expression cassette including a promoter, a gene encoding a target protein, and a gene of a hairpin structure at the 3'-terminus thereof, or an expression vector including the expression cassette; and
(ii) culturing the transformant.

15. The method of claim 14, further comprising the step of (iii) collecting the target protein from the transformant or a culture thereof.


<110>     Intelligent Synthetic Biology Center

<120>     A composition for solubilization of target proteins and use
          thereof

<130>     I33142EP

<160>     79

<170>     KoPatentIn 3.0

<210>     1
<211>     54
<212>     DNA
<213>     Artificial Sequence

<220>
<223>     DnaJNcoF


<400>     1
tgataaccat ggaagattct acggttaaca caatggctaa gcaagattat tacg              54


<210>     2
<211>     30
<212>     DNA
<213>     Artificial Sequence

<220>
<223>     DnaJXhoR


<400>     2
atttcactcg aggcgggtca ggtcgtcaaa                                         30


<210>     3
<211>     94
<212>     DNA
<213>     Artificial Sequence

<220>
<223>     KHNcoF1


<400>     3
taatgaccat ggaaaccgac ggttctaaag acgttgttga aatcgctgtt ccggaaaacc        60

tggttggtgc tatcctgggt aaaggtggta aaac                                    94


<210>     4
<211>     92
<212>     DNA
<213>     Artificial Sequence

<220>
<223>     KHRecR1


<400>     4
gcccggaaca aattcacctt ttttagaaat ctggatacga gcacctgtca gttcctggta        60

ttcaaccagg gttttaccac ctttacccag ga                                    92

<210>    5
<211>    92
<212>    DNA
<213>    Artificial Sequence

<220>
<223>    KHRecF2

<400>    5
aaaaggtgaa tttgttccgg gcacccgtaa ccgtaaagtt accatcacag gcaccccggc      60

tgctacccag gctgctcagt acctgatcac ac                                    92

<210>    6
<211>    90
<212>    DNA
<213>    Artificial Sequence

<220>
<223>    KHXhoR2

<400>    6
ttatcactcg agttaaccaa ctttctgcgg gttagcagca cgaacaccct gttcgtaggt      60

gatacgctgt gtgatcaggt actgagcagc                                       90

<210>    7
<211>    48
<212>    DNA
<213>    Artificial Sequence

<220>
<223>    DnaJLinkR

<400>    7
gctgccgcca ccaccgctac cgccaccgcc gcgggtcagg tcgtcaaa                   48

<210>    8
<211>    42
<212>    DNA
<213>    Artificial Sequence

<220>
<223>    LinkKHF

<400>    8
ggtagcggtg gtggcggcag caccgacggt tctaaagacg tt                         42

<210>    9
<211>    33
<212>    DNA

<213>    Artificial Sequence

<220>
<223>    KHXhoR


<400>    9
atttcactcg agttaaccaa ctttctgcgg gtt                                    33


<210>    10
<211>    77
<212>    DNA
<213>    Artificial Sequence

<220>
<223>    KH-6xHis-EcoR


<400>    10
cgattaggat cctcatcatt aatgatggtg gtgatggtga gatccacgcg gaaccagacc       60

aactttctgc gggttag                                                      77


<210>    11
<211>    51
<212>    DNA
<213>    Artificial Sequence

<220>
<223>    DnaJR


<400>    11
agaacctccg ccgccagaac ccccgccacc gcgggtcagg tcgtcaaaaa a                 51


<210>    12
<211>    43
<212>    DNA
<213>    Artificial Sequence

<220>
<223>    DnaKF


<400>    12
tctggcggcg gaggttctgg taaaataatt ggtatcgacc tgg                         43


<210>    13
<211>    42
<212>    DNA
<213>    Artificial Sequence

<220>
<223>    DnaKR


<400>    13
gctaccgcca ccgccttttt tgtctttgac ttcttcaaat tc                          42

<210>    14
<211>    24
<212>    DNA
<213>    Artificial Sequence

<220>
<223>    KHF


<400>    14
gacaaaaaag gcggtggcgg tagc                                              24


<210>    15
<211>    28
<212>    DNA
<213>    Artificial Sequence

<220>
<223>    ScFvNdeF


<400>    15
tgataacata tgcaggtcca actgcagc                                          28


<210>    16
<211>    59
<212>    DNA
<213>    Artificial Sequence

<220>
<223>    ScFvXhoR


<400>    16
tcattactcg agtcatcatt agtggtggtg gtggtggtgt ttgatctcca gcttggtcc        59


<210>    17
<211>    96
<212>    DNA
<213>    Artificial Sequence

<220>
<223>    ScFv1LXhoR


<400>    17
ccgttactcg agccgcgcgg ggtgatctag gtccgcgcgg tcgtcgtcgt catcattagt       60

ggtggtggtg gtggtgtttg atctccagct tggtcc                                 96


<210>    18
<211>    80
<212>    DNA
<213>    Artificial Sequence

<220>
<223>    3L-1R

<400>    18
aggtgagcaa cggacatcct tcacgggtga tctaggtcgt gaaggctcga tcgtcatcat    60

tagtggtggt ggtggtggtg                                                80


<210>    19
<211>    87
<212>    DNA
<213>    Artificial Sequence

<220>
<223>    3LXhoR


<400>    19
ccgttactcg agtcgtagag cggtgatcta ggtgctctac ggactgcgtt gctcggtgat    60

ctaggtgagc aacggacatc cttcacg                                        87


<210>    20
<211>    28
<212>    DNA
<213>    Artificial Sequence

<220>
<223>    BR2ScFvNdeF


<400>    20
tgataacata tgcgtgctgg tctgcagt                                       28


<210>    21
<211>    28
<212>    DNA
<213>    Artificial Sequence

<220>
<223>    BR2ScFvNdeF


<400>    21
tgataacata tgcgtgctgg tctgcagt                                       28


<210>    22
<211>    29
<212>    DNA
<213>    Artificial Sequence

<220>
<223>    UGDNdeF


<400>    22
tgataacata tgaaaatcac catttccgg                                      29


<210>    23
<211>    55

<212>     DNA
<213>     Artificial Sequence

<220>
<223>     UGDXhoR


<400>     23
ccgttactcg agttattagt ggtggtggtg gtggtggtcg ctgccaaaga gatcg          55


<210>     24
<211>     95
<212>     DNA
<213>     Artificial Sequence

<220>
<223>     UGD1LXhoR


<400>     24
ccgttactcg agccgcgcgg ggtgatctag gtccgcgcgg tcgtcgtcgt catcattagt     60

ggtggtggtg gtggtggtcg ctgccaaaga gatcg                                95


<210>     25
<211>     32
<212>     DNA
<213>     Artificial Sequence

<220>
<223>     AdhNdeF


<400>     25
tgataacata tgtctatccc agaaactcaa aa                                   32


<210>     26
<211>     60
<212>     DNA
<213>     Artificial Sequence

<220>
<223>     AdhXhoR


<400>     26
ccgttactcg agttattagt ggtggtggtg gtggtgttta gaagtgtcaa caacgtatct     60

                                                                      60


<210>     27
<211>     100
<212>     DNA
<213>     Artificial Sequence

<220>
<223>     Adh1LXhoR

<400>    27
ccgttactcg agccgcgcgg ggtgatctag gtccgcgcgg tcgtcgtcgt catcattagt    60

ggtggtggtg gtggtgttta gaagtgtcaa caacgtatct                          100


<210>    28
<211>    80
<212>    DNA
<213>    Artificial Sequence

<220>
<223>    3L-1R


<400>    28
aggtgagcaa cggacatcct tcacgggtga tctaggtcgt gaaggctcga tcgtcatcat    60

tagtggtggt ggtggtggtg                                                80


<210>    29
<211>    87
<212>    DNA
<213>    Artificial Sequence

<220>
<223>    3LXhoR


<400>    29
ccgttactcg agtcgtagag cggtgatcta ggtgctctac ggactgcgtt gctcggtgat    60

ctaggtgagc aacggacatc cttcacg                                        87


<210>    30
<211>    33
<212>    DNA
<213>    Artificial Sequence

<220>
<223>    UbiCNdeF


<400>    30
tgataacata tgcgattgtt gcgtttttgt tgc                                 33


<210>    31
<211>    56
<212>    DNA
<213>    Artificial Sequence

<220>
<223>    UbiCXhoR


<400>    31
ccgttactcg agttattagt ggtggtggtg gtggtggtac aacggtgacg ccggta        56


<210>    32

<211>	96
<212>	DNA
<213>	Artificial Sequence

<220>
<223>	UbiC1LXhoR


<400>	32
ccgttactcg agccgcgcgg ggtgatctag gtccgcgcgg tcgtcgtcgt catcattagt	60

ggtggtggtg gtggtggtac aacggtgacg ccggta	96


<210>	33
<211>	80
<212>	DNA
<213>	Artificial Sequence

<220>
<223>	3L-1R


<400>	33
aggtgagcaa cggacatcct tcacgggtga tctaggtcgt gaaggctcga tcgtcatcat	60

tagtggtggt ggtggtggtg	80


<210>	34
<211>	87
<212>	DNA
<213>	Artificial Sequence

<220>
<223>	3LXhoR


<400>	34
ccgttactcg agtcgtagag cggtgatcta ggtgctctac ggactgcgtt gctcggtgat	60

ctaggtgagc aacggacatc cttcacg	87


<210>	35
<211>	83
<212>	DNA
<213>	Artificial Sequence

<220>
<223>	HIVPrXbaF


<400>	35
attctaaatc tagattattc actacgcgtt aaggaggtac gacatgcacc atcaccacca	60

tcatcctcaa atcaccctgt ggc	83


<210>	36
<211>	85
<212>	DNA
<213>	Artificial Sequence

<220>
<223>    HIVPrXhoR

<400>    36
ccgaattact cgagtcatca ttagaagttc agggtgcaac cgatctgggt cagcatgtta    60

cgaccgatga tgttgatcgg ggtcg                                          85

<210>    37
<211>    77
<212>    DNA
<213>    Artificial Sequence

<220>
<223>    HIVPr1LXhoR

<400>    37
ccgttactcg agccgcgcgg ggtgatctag gtccgcgcgg tcgtcgtcgt catcattaga    60

agttcagggt gcaaccg                                                   77

<210>    38
<211>    78
<212>    DNA
<213>    Artificial Sequence

<220>
<223>    HIVPrXho3LR

<400>    38
ccgaattact cgagccgcgc ggggtgatct aggtccgcgc ggtcgtcgtc gtcatcatta    60

gaagttcagg gtgcaacc                                                  78

<210>    39
<211>    33
<212>    DNA
<213>    Artificial Sequence

<220>
<223>    LepNdeF

<400>    39
tgataacata tgcgattgtt gcgtttttgt tgc                                 33

<210>    40
<211>    56
<212>    DNA
<213>    Artificial Sequence

<220>
<223>    LepXhoR

<400>    40
ccgttactcg agttattagt ggtggtggtg gtggtggtac aacggtgacg ccggta          56

<210>    41
<211>    96
<212>    DNA
<213>    Artificial Sequence

<220>
<223>    Lep1LXhoR

<400>    41
ccgttactcg agccgcgcgg ggtgatctag gtccgcgcgg tcgtcgtcgt catcattagt      60

ggtggtggtg gtggtggtac aacggtgacg ccggta                                96

<210>    42
<211>    80
<212>    DNA
<213>    Artificial Sequence

<220>
<223>    3L-1R

<400>    42
aggtgagcaa cggacatcct tcacgggtga tctaggtcgt gaaggctcga tcgtcatcat      60

tagtggtggt ggtggtggtg                                                  80

<210>    43
<211>    87
<212>    DNA
<213>    Artificial Sequence

<220>
<223>    3LXhoR

<400>    43
ccgttactcg agtcgtagag cggtgatcta ggtgctctac ggactgcgtt gctcggtgat      60

ctaggtgagc aacggacatc cttcacg                                          87

<210>    44
<211>    30
<212>    DNA
<213>    Artificial Sequence

<220>
<223>    BMP2NdeF

<400>    44
tgataacata tgcaagccaa acacaaacag                                       30

<210>    45

<211>    54
<212>    DNA
<213>    Artificial Sequence

<220>
<223>    BMP2XhoR


<400>    45
ccgttactcg agttattagt ggtggtggtg gtggtggcga cacccacaac cctc          54


<210>    46
<211>    93
<212>    DNA
<213>    Artificial Sequence

<220>
<223>    BMP21LXhoR


<400>    46
ccgttactcg agccgcgcgg ggtgatctag gtccgcgcgg tcgtcgtcgt catcattagt    60

ggtggtggtg gtggtgcgac acccacaacc ctc                                 93


<210>    47
<211>    80
<212>    DNA
<213>    Artificial Sequence

<220>
<223>    3L-1R


<400>    47
aggtgagcaa cggacatcct tcacgggtga tctaggtcgt gaaggctcga tcgtcatcat    60

tagtggtggt ggtggtggtg                                                80


<210>    48
<211>    30
<212>    DNA
<213>    Artificial Sequence

<220>
<223>    sfGFPNdeF


<400>    48
tgataacata tgcaagccaa acacaaacag                                     30


<210>    49
<211>    40
<212>    DNA
<213>    Artificial Sequence

<220>
<223>    sfGFPEcoR

<400>    49
aggtcagaat tctcatcatt acgtaatacc tgccgcattc                              40


<210>    50
<211>    68
<212>    DNA
<213>    Artificial Sequence

<220>
<223>    CsfGFPXbaF


<400>    50
ccatgatcta gaaataattt tgtttaactt taagaaggag atataccatg gtgcccatcc        60

aaaaagtc                                                                 68


<210>    51
<211>    42
<212>    DNA
<213>    Artificial Sequence

<220>
<223>    CsfGFPNdeR


<400>    51
ccgttacata tgtcatcatt atgtaatccc agcagcattt ac                           42


<210>    52
<211>    30
<212>    DNA
<213>    Artificial Sequence

<220>
<223>    sfGFPNdeF


<400>    52
tgataacata tgcaagccaa acacaaacag                                         30


<210>    53
<211>    44
<212>    DNA
<213>    Artificial Sequence

<220>
<223>    sfNGFPEcoR


<400>    53
aggtcagaat tctcatcatt atttttcgtt cggatcttta gaca                         44


<210>    54
<211>    85
<212>    DNA
<213>    Artificial Sequence

<220>
<223>    sfNGFP3L-1R


<400>    54
aggtgagcaa cggacatcct tcacgggtga tctaggtcgt gaaggctcga tcgtcatcat      60

tatttttcgt tcggatcttt agaca                                            85


<210>    55
<211>    86
<212>    DNA
<213>    Artificial Sequence

<220>
<223>    sfNGFP3LEcoR


<400>    55
aggtcagaat tctcgtagag cggtgatcta ggtgctctac ggactgcgtt gctcggtgat      60

ctaggtgagc aacggacatc cttcac                                           86


<210>    56
<211>    31
<212>    DNA
<213>    Artificial Sequence

<220>
<223>    DnaJNdeF


<400>    56
tgataacata tggctaagca agattattac g                                     31


<210>    57
<211>    55
<212>    DNA
<213>    Artificial Sequence

<220>
<223>    ScFvFpol


<400>    57
gttttttgac gacctgaccc gcgaggaggt ggaataatgc aggtccaact gcagc          55


<210>    58
<211>    54
<212>    DNA
<213>    Artificial Sequence

<220>
<223>    DnaJFpol


<400>    58
caccaccacc accaccacga ggaggtggaa taatggctaa gcaagattat tacg            54

<210>    59
<211>    60
<212>    DNA
<213>    Artificial Sequence

<220>
<223>    UbiCFpol


<400>    59
gttttttgac gacctgaccc gcgaggaggt ggaataatgc gattgttgcg tttttgttgc        60

                                                                          60


<210>    60
<211>    76
<212>    DNA
<213>    Artificial Sequence

<220>
<223>    HIVPrFpol


<400>    60
gttttttgac gacctgaccc gcgaggaggt ggaataatgc accatcacca ccatcatcct        60

caaatcaccc tgtggc                                                       76


<210>    61
<211>    55
<212>    DNA
<213>    Artificial Sequence

<220>
<223>    DnaJFpolHIVPr


<400>    61
cggttgcacc ctgaacttcg aggaggtgga ataatggcta agcaagatta ttacg            55


<210>    62
<211>    60
<212>    DNA
<213>    Artificial Sequence

<220>
<223>    LepFpol


<400>    62
gttttttgac gacctgaccc gcgaggaggt ggaataatgc gattgttgcg tttttgttgc        60

                                                                          60


<210>    63
<211>    57
<212>    DNA

<213>    Artificial Sequence

<220>
<223>    BMP2FPol


<400>    63
   gttttttgac gacctgaccc gcgaggaggt ggaataatgc aagccaaaca caaacag          57


<210>    64
<211>    59
<212>    DNA
<213>    Artificial Sequence

<220>
<223>    AdhFPol


<400>    64
   gttttttgac gacctgaccc gcgaggaggt ggaataatgt ctatcccaga aactcaaaa        59


<210>    65
<211>    43
<212>    DNA
<213>    Artificial Sequence

<220>
<223>    TliANdeF


<400>    65
tgataacata tgcatcatca tcatcatcat catcatcaca gca                        43


<210>    66
<211>    42
<212>    DNA
<213>    Artificial Sequence

<220>
<223>    TliAEcoR


<400>    66
ctgagaattc tcatcattaa ctgatcagca caccctcgct cc                         42


<210>    67
<211>    70
<212>    DNA
<213>    Artificial Sequence

<220>
<223>    TliAFPol


<400>    67
   gttttttgac gacctgaccc gcgaggaggt ggaataatgc atcatcatca tcatcatcat       60
   catcacagca                                                             70

<210>    68
<211>    59
<212>    DNA
<213>    Artificial Sequence

<220>
<223>    DnaJTliAFpol


<400>    68
ggagcgaggg tgtgctgatc agtgaggagg tggaataatg gctaagcaag attattacg         59


<210>    69
<211>    34
<212>    DNA
<213>    Artificial Sequence

<220>
<223>    DnaJEcoR


<400>    69
ctgagaattc ttattagcgg gtcaggtcgt caaa                                    34


<210>    70
<211>    39
<212>    DNA
<213>    Artificial Sequence

<220>
<223>    TliA1EcoR


<400>    70
ccgttagaat tctcatcatt agtcggtggt cgactcgtg                               39


<210>    71
<211>    71
<212>    DNA
<213>    Artificial Sequence

<220>
<223>    TliA1FPol


<400>    71
agttttttga cgacctgacc cgcaccggag gtacataatg catcatcatc atcatcatca         60

tcatcacagc a                                                             71


<210>    72
<211>    39
<212>    DNA
<213>    Artificial Sequence

<220>
<223>    TliA1EcoR

<400>     72
ccgttagaat tctcatcatt agtcggtggt cgactcgtg                              39


<210>     73
<211>     31
<212>     DNA
<213>     Artificial Sequence

<220>
<223>     TliA2NdeF


<400>     73
tgataacata tgaacatcgt cagcttcaac g                                      31


<210>     74
<211>     1131
<212>     DNA
<213>     Escherichia coli K-12

<400>     74
atggctaagc aagattatta cgagatttta ggcgtttcca aaacagcgga agagcgtgaa       60

atcagaaagg cctacaaacg cctggccatg aaataccacc cggaccgtaa ccagggtgac      120

aaagaggccg aggcgaaatt taaagagatc aaggaagctt atgaagttct gaccgactcg      180

caaaaacgtg cggcatacga tcagtatggt catgctgcgt ttgagcaagg tggcatgggc      240

ggcggcggtt ttggcggcgg cgcagacttc agcgatattt ttggtgacgt tttcggcgat      300

atttttggcg gcggacgtgg tcgtcaacgt gcggcgcgcg gtgctgattt acgctataac      360

atggagctca ccctcgaaga agctgtacgt ggcgtgacca aagagatccg cattccgact      420

ctggaagagt gtgacgtttg ccacggtagc ggtgcaaaac caggtacaca gccgcagact      480

tgtccgacct gtcatggttc tggtcaggtg cagatgcgcc agggattctt cgctgtacag      540

cagacctgtc cacactgtca gggccgcggt acgctgatca aagatccgtg caacaaatgt      600

catggtcatg gtcgtgttga gcgcagcaaa acgctgtccg ttaaaatccc ggcaggggtg      660

gacactggag accgcatccg tcttgcgggc gaaggtgaag cgggcgagca tggcgcaccg      720

gcaggcgatc tgtacgttca ggttcaggtt aaacagcacc cgattttcga gcgtgaaggc      780

aacaacctgt attgcgaagt cccgatcaac ttcgctatgg cggcgctggg tggcgaaatc      840

gaagtaccga cccttgatgg tcgcgtcaaa ctgaaagtgc ctggcgaaac ccagaccggt      900

aagctattcc gtatgcgcgg taaaggcgtc aagtctgtcc gcggtggcgc acagggtgat      960

ttgctgtgcc gcgttgtcgt cgaaacaccg gtaggcctga acgaaaggca gaaacagctg     1020

ctgcaagagc tgcaagaaag cttcggtggc ccaaccggcg agcacaacag cccgcgctca     1080

aagagcttct ttgatggtgt gaagaagttt tttgacgacc tgacccgcta a              1131

<210>    75
<211>    1917
<212>    DNA
<213>    Escherichia coli K-12

<400>    75
atgggtaaaa taattggtat cgacctgggt actaccaact cttgtgtagc gattatggat    60

ggcaccactc ctcgcgtgct ggagaacgcc gaaggcgatc gcaccacgcc ttctatcatt    120

gcctataccc aggatggtga aactctagtt ggtcagccgg ctaaacgtca ggcagtgacg    180

aacccgcaaa acactctgtt tgcgattaaa cgcctgattg gtcgccgctt ccaggacgaa    240

gaagtacagc gtgatgtttc catcatgccg ttcaaaatta ttgctgctga taacggcgac    300

gcatgggtcg aagttaaagg ccagaaaatg gcaccgccgc agatttctgc tgaagtgctg    360

aaaaaaatga agaaaaccgc tgaagattac ctgggtgaac cggtaactga agctgttatc    420

accgtaccgg catactttaa cgatgctcag cgtcaggcaa ccaaagacgc aggccgtatc    480

gctggtctgg aagtaaaacg tatcatcaac gaaccgaccg cagctgcgct ggcttacggt    540

ctggacaaag gcactggcaa ccgtactatc gcggtttatg acctgggtgg tggtactttc    600

gatatttcta ttatcgaaat cgacgaagtt gacggcgaaa aaaccttcga agttctggca    660

accaacggtg atacccacct ggggggtgaa gacttcgaca gccgtctgat caactatctg    720

gttgaagaat tcaagaaaga tcagggcatt gacctgcgca acgatccgct ggcaatgcag    780

cgcctgaaag aagcggcaga aaaagcgaaa atcgaactgt cttccgctca gcagaccgac    840

gttaacctgc catacatcac tgcagacgcg accggtccga aacacatgaa catcaaagtg    900

actcgtgcga aactggaaag cctggttgaa gatctggtaa accgttccat tgagccgctg    960

aaagttgcac tgcaggacgc tggcctgtcc gtatctgata tcgacgacgt tatcctcgtt    1020

ggtggtcaga ctcgtatgcc aatggttcag aagaaagttg ctgagttctt tggtaaagag    1080

ccgcgtaaag acgttaaccc ggacgaagct gtagcaatcg gtgctgctgt tcagggtggt    1140

gttctgactg gtgacgtaaa agacgtactg ctgctggacg ttaccccgct gtctctgggt    1200

atcgaaacga tgggcggtgt gatgacgacg ctgatcgcga aaaacaccac tatcccgacc    1260

aagcacagcc aggtgttctc taccgctgaa gacaaccagt ctgcggtaac catccatgtg    1320

ctgcagggtg aacgtaaacg tgcggctgat aacaaatctc tgggtcagtt caacctagat    1380

ggtatcaacc cggcaccgcg cggcatgccg cagatcgaag ttaccttcga tatcgatgct    1440

gacggtatcc tgcacgtttc cgcgaaagat aaaaacagcg gtaaagagca gaagatcacc    1500

atcaaggctt cttctggtct gaacgaagat gaaatccaga aaatggtacg cgacgcagaa    1560

gctaacgccg aagctgaccg taagtttgaa gagctggtac agactcgcaa ccagggcgac    1620

catctgctgc acagcacccg taagcaggtt gaagaagcag gcgacaaact gccggctgac    1680

gacaaaactg ctatcgagtc tgcgctgact gcactggaaa ctgctctgaa aggtgaagac    1740

aaagccgcta tcgaagcgaa aatgcaggaa ctggcacagg tttcccagaa actgatggaa 1800

atcgcccagc agcaacatgc ccagcagcag actgccggtg ctgatgcttc tgcaaacaac 1860

gcgaaagatg acgatgttgt cgacgctgaa tttgaagaag tcaaagacaa aaaataa 1917

<210> 76
<211> 276
<212> DNA
<213> Artificial Sequence

<220>
<223> Nova-1 KH3 RNA binding domain

<400> 76
accgacggtt ctaaagacgt tgttgaaatc gctgttccgg aaaacctggt tggtgctatc 60

ctgggtaaag gtggtaaaac cctggttgaa taccaggaac tgacaggtgc tcgtatccag 120

atttctaaaa aaggtgaatt tgttccgggc acccgtaacc gtaaagttac catcacaggc 180

accccggctg ctacccaggc tgctcagtac ctgatcacac agcgtatcac ctacgaacag 240

ggtgttcgtg ctgctaaccc gcagaaagtt ggttaa 276

<210> 77
<211> 12
<212> DNA
<213> Artificial Sequence

<220>
<223> loop having the binding site for KH RNA binding domain

<400> 77
acctagatca cc 12

<210> 78
<211> 4
<212> PRT
<213> Artificial Sequence

<220>
<223> a region introduced into the C-terminus of first cistron

<400> 78
Glu Glu Val Glu
1

<210> 79
<211> 12
<212> DNA
<213> Artificial Sequence

<220>
<223> a region introduced into the C-terminus of first cistron

<400>    79
gaggaggtgg aa                                                          12